(19) Europäisches Patentamt
European Patent Office
Office européen des brevets

(11) EP 1 590 346 B1

(12) **EUROPEAN PATENT SPECIFICATION**

(45) Date of publication and mention of the grant of the patent:
**25.10.2006 Bulletin 2006/43**

(21) Application number: **04701628.2**

(22) Date of filing: **13.01.2004**

(51) Int Cl.:
***C07D 409/06*** *(2006.01)* ***C07D 405/06*** *(2006.01)*
***C07D 211/70*** *(2006.01)* ***C07D 401/06*** *(2006.01)*
***C07D 417/06*** *(2006.01)* ***C07C 311/08*** *(2006.01)*
***A61K 31/4436*** *(2006.01)* ***A61K 31/443*** *(2006.01)*
***A61K 31/44*** *(2006.01)* ***A61K 31/444*** *(2006.01)*
***A61K 31/4439*** *(2006.01)* ***A61P 25/04*** *(2006.01)*
***A61P 25/22*** *(2006.01)* ***A61P 1/04*** *(2006.01)*

(86) International application number:
**PCT/GB2004/000061**

(87) International publication number:
**WO 2004/063193 (29.07.2004 Gazette 2004/31)**

(54) **4- {'3-(SULFONYLAMINO) PHENYL] '1-(CYCLYMETHYL) PIPERIDIN-4-YLIDENE] METHYL} BENAZMIDE DERIVATIVES AS DELTA OPIOID RECEPTOR LIGANDS FOR THE TREATMENT OF PAIN, ANXIETY AND FUNCTIONAL GASTROINTESTINAL DISORDER**

4-{'3-(SULFONYLAMINO)PHENYL]'1-(CYCLYMETHYL)PIPERIDIN-4-YLIDENE]METHYL} BENZAMIDEDERIVATE ALS DELTA-OPIOID-REZEPTORLIGANDEN ZUR BEHANDLUNG VON SCHMERZEN,ANGSTZUSTÄNDEN UND FUNKTIONELLEN GASTROINTESTINALEN ERKRANKUNGEN

DERIVES DE 4-{'3-(SULFONYLAMINO)PHENYL]'1-(CYCLYMETHYL)PIPERIDIN-4-YLIDENE] METHYL} BENZAMIDE UTILISES COMME LIGANDS DES RECEPTEURS DES OPIOIDES DELTA POUR LE TRAITEMENT DE LA DOULEUR, DE L'ANXIETE ET DE TROUBLES FONCTIONNELS GASTRO-INTESTINAUX

(84) Designated Contracting States:
**AT BE BG CH CY CZ DE DK EE ES FI FR GB GR HU IE IT LI LU MC NL PT RO SE SI SK TR**

(30) Priority: **16.01.2003 SE 0300104**

(43) Date of publication of application:
**02.11.2005 Bulletin 2005/44**

(73) Proprietor: **AstraZeneca AB**
**151 85 (SE)**

(72) Inventors:
- **BROWN, William,**
**AstraZeneca R & D Montreal**
**St Laurent,**
**Québec H4S 1Z9 (CA)**
- **GRIFFIN, Andrew,**
**AstraZeneca R & D Montreal**
**St. Laurent,**
**Québec H4S 1Z9 (CA)**
- **WALPOLE, Christopher,**
**AstraZeneca R & D Montreal**
**St. Laurent,**
**Quebec H4S 1Z9 (CA)**

(56) References cited:
**WO-A-93/15062** **WO-A-98/28275**
**WO-A-02/094786** **WO-A-02/094812**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

The present invention is directed to novel compounds, processes for their preparation, their uses and pharmaceutical compositions comprising the novel compounds. The novel compounds are useful in therapy, and in particular for the treatment of pain, anxiety and functional gastrointestinal disorders.

### BACKGROUND OF THE INVENTION

The receptor has been identified as having a role in many bodily functions such as circulatory and pain systems. Ligands for the δ receptor may therefore find potential use as analgesics, and/or as antihypertensive agents. Ligands for the δ receptor have also been shown to possess immunomodulatory activities.

The identification of at least three different populations of opioid receptors (μ, δ and κ) is now well established and all three are apparent in both central and peripheral nervous systems of many species including man. Analgesia has been observed in various animal models when one or more of these receptors has been activated.

With few exceptions, currently available selective opioid δ ligands are peptidic in nature and are unsuitable for administration by systemic routes. One example of a non-peptidic δ-agonist is SNC80 (Bilsky E.J. et al., Journal of Pharmacology and Experimental Therapeutics, 273(1), pp. 359-366 (1995)).

Many δ agonist compounds that have been identified in the prior art have many disadvantages in that they suffer from poor pharmacokinetics and are not analgesic when administered by systemic routes. Also, it has been documented that many of these δ agonist compounds show significant convulsive effects when administered systemically.

U.S. Patent No. 6,187,792 to Delorme et al. describes some δ-agonists.

However, there is still a need for improved δ-agonists.

### DESCRIPTION OF THE INVENTION

Unless specified otherwise within this specification, the nomenclature used in this specification generally follows the examples and rules stated in *Nomenclature of Organic Chemistry, Sections A, B, C, D, E, F, and H,* Pergamon Press, Oxford, 1979, which is incorporated by references herein for its exemplary chemical structure names and rules on naming chemical structures. Optionally, a name of a compound may be generated using a chemical naming program: ACD/ChemSketch, Version 5.09/September 2001, Advanced Chemistry Development, Inc., Toronto, Canada.

The term "$C_{m-n}$" or "$C_{m-n}$ group" used alone or as a prefix, refers to any group having m to n carbon atoms.

The term "hydrocarbon" used alone or as a suffix or prefix, refers to any structure comprising only carbon and hydrogen atoms up to 14 carbon atoms.

The term "hydrocarbon radical" or "hydrocarbyl" used alone or as a suffix or prefix, refers to any structure as a result of removing one or more hydrogens from a hydrocarbon.

The term "alkyl" used alone or as a suffix or prefix, refers to monovalent straight or branched chain hydrocarbon radicals comprising 1 to about 12 carbon atoms. Unless otherwise specified, "alkyl" general includes both saturated alkyl and unsaturated alkyl.

The term "alkylene" used alone or as suffix or prefix, refers to divalent straight or branched chain hydrocarbon radicals comprising 1 to about 12 carbon atoms, which serves to links two structures together.

The term "alkenyl" used alone or as suffix or prefix, refers to a monovalent straight or branched chain hydrocarbon radical having at least one carbon-carbon double bond and comprising at least 2 up to about 12 carbon atoms.

The term "alkynyl" used alone or as suffix or prefix, refers to a monovalent straight or branched chain hydrocarbon radical having at least one carbon-carbon triple bond and comprising at least 2 up to about 12 carbon atoms.

The term "cycloalkyl," used alone or as suffix or prefix, refers to a monovalent ring-containing hydrocarbon radical comprising at least 3 up to about 12 carbon atoms.

The term "cycloalkenyl" used alone or as suffix or prefix, refers to a monovalent ring-containing hydrocarbon radical having at least one carbon-carbon double bond and comprising at least 3 up to about 12 carbon atoms.

The term "cycloalkynyl" used alone or as suffix or prefix, refers to a monovalent ring-containing hydrocarbon radical having at least one carbon-carbon triple bond and comprising about 7 up to about 12 carbon atoms.

The term "aryl" used alone or as suffix or prefix, refers to a monovalent hydrocarbon radical having one or more polyunsaturated carbon rings having aromatic character, (*e.g.,* 4n + 2 delocalized electrons) and comprising 5 up to about 14 carbon atoms.

The term "arylene" used alone or as suffix or prefix, refers to a divalent hydrocarbon radical having one or more polyunsaturated carbon rings having aromatic character, (*e.g.*, 4n + 2 delocalized electrons) and comprising 5 up to about 14 carbon atoms, which serves to links two structures together.

The term "heterocycle" used alone or as a suffix or prefix, refers to a ring-containing structure or molecule having one or more multivalent heteroatoms, independently selected from N, O and S, as a part of the ring structure and including at least 3 and up to about 20 atoms in the ring(s). Heterocycle may be saturated or unsaturated, containing one or more double bonds, and heterocycle may contain more than one ring. When a heterocycle contains more than one ring, the rings may be fused or unfused. Fused rings generally refer to at least two rings share two atoms therebetween. Heterocycle may have aromatic character or may not have aromatic character.

The term "heteroalkyl" used alone or as a suffix or prefix, refers to a radical formed as a result of replacing one or more carbon atom of an alkyl with one or more heteroatoms selected from N, O and S.

The term "heteroaromatic" used alone or as a suffix or prefix, refers to a ring-containing structure or molecule having one or more multivalent heteroatoms, independently selected from N, O and S, as a part of the ring structure and including at least 3 and up to about 20 atoms in the ring(s), wherein the ring-containing structure or molecule has an aromatic character (*e.g.*, 4n + 2 delocalized electrons).

The term "heterocyclic group," "heterocyclic moiety," "heterocyclic," or "heterocyclo" used alone or as a suffix or prefix, refers to a radical derived from a heterocycle by removing one or more hydrogens therefrom.

The term "heterocyclyl" used alone or as a suffix or prefix, refers a monovalent radical derived from a heterocycle by removing one hydrogen therefrom.

The term "heterocyclylene" used alone or as a suffix or prefix, refers to a divalent radical derived from a heterocycle by removing two hydrogens therefrom, which serves to links two structures together.

The term "heteroaryl" used alone or as a suffix or prefix, refers to a heterocyclyl having aromatic character.

The term "heterocylcoalkyl" used alone or as a suffix or prefix, refers to a heterocyclyl that does not have aromatic character.

The term "heteroarylene" used alone or as a suffix or prefix, refers to a heterocyclylene having aromatic character.

The term "heterocycloalkylene" used alone or as a suffix or prefix, refers to a heterocyclylene that does not have aromatic character.

The term "six-membered" used as prefix refers to a group having a ring that contains six ring atoms.

The term "five-membered" used as prefix refers to a group having a ring that contains five ring atoms.

A five-membered ring heteroaryl is a heteroaryl with a ring having five ring atoms wherein 1, 2 or 3 ring atoms are independently selected from N, O and S.

Exemplary five-membered ring heteroaryls are thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4- oxadiazolyl.

A six-membered ring heteroaryl is a heteroaryl with a ring having six ring atoms wherein 1, 2 or 3 ring atoms are independently selected from N, O and S.

Exemplary six-membered ring heteroaryls are pyridyl, pyrazinyl, pyrimidinyl, triazinyl and pyridazinyl.

The term "substituted" used as a prefix refers to a structure, molecule or group, wherein one or more hydrogens are replaced with one or more $C_{1-12}$hydrocarbon groups, or one or more chemical groups containing one or more heteroatoms selected from N, O, S, F, Cl, Br, I, and P. Exemplary chemical groups containing one or more heteroatoms include heterocyclyl, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R,-CN, -OH, -C(=O)OR, $-C(=O)NR_2$, -NRC(=O)R, oxo (=O), imino (=NR), thio (=S), and oximino (=N-OR), wherein each "R" is a $C_{1-12}$hydrocarbyl. For example, substituted phenyl may refer to nitrophenyl, pyridylphenyl, methoxyphenyl, chlorophenyl, aminophenyl, etc., wherein the nitro, pyridyl, methoxy, chloro, and amino groups may replace any suitable hydrogen on the phenyl ring.

The term "substituted" used as a suffix of a first structure, molecule or group, followed by one or more names of chemical groups refers to a second structure, molecule or group, which is a result of replacing one or more hydrogens of the first structure, molecule or group with the one or more named chemical groups. For example, a "phenyl substituted by nitro" refers to nitrophenyl.

The term "optionally substituted" refers to both groups, structures, or molecules that are substituted and those that are not substituted.

Heterocycle includes, for example, monocyclic heterocycles such as: aziridine, oxirane, thiirane, azetidine, oxetane, thietane, pyrrolidine, pyrroline, imidazolidine, pyrazolidine, pyrazoline, dioxolane, sulfolane 2,3-dihydrofuran, 2,5-dihydrofuran tetrahydrofuran, thiophane, piperidine, 1,2,3,6-tetrahydro-pyridine, piperazine, morpholine, thiomorpholine, pyran, thiopyran, 2,3-dihydropyran, tetrahydropyran, 1,4-dihydropyridine, 1,4-dioxane, 1,3-dioxane, dioxane, homopiperidine, 2,3,4,7-tetrahydro-1*H*-azepine homopiperazine, 1,3-dioxepane, 4,7-dihydro-1,3-dioxepin, and hexamethylene oxide.

In addition, heterocycle includes aromatic heterocycles, for example, pyridine, pyrazine, pyrimidine, pyridazine, thiophene, furan, furazan, pyrrole, imidazole, thiazole, oxazole, pyrazole, isothiazole, isoxazole, 1,2,3-triazole, tetrazole, 1,2,3-thiadiazole, 1,2,3-oxadiazole, 1,2,4-triazole, 1,2,4-thiadiazole, 1,2,4-oxadiazole, 1,3,4-triazole, 1,3,4-thiadiazole,

and 1,3,4- oxadiazole.

Additionally, heterocycle encompass polycyclic heterocycles, for example, indole, indoline, isoindoline, quinoline, tetrahydroquinoline, isoquinoline, tetrahydroisoquinoline, 1,4-benzodioxan, coumarin, dihydrocoumarin, benzofuran, 2,3-dihydrobenzofuran, isobenzofuran, chromene, chroman, isochroman, xanthene, phenoxathiin, thianthrene, indolizine, isoindole, indazole, purine, phthalazine, naphthyridine, quinoxaline, quinazoline, cinnoline, pteridine, phenanthridine, perimidine, phenanthroline, phenazine, phenothiazine, phenoxazine, 1,2-benzisoxazole, benzothiophene, benzoxazole, benzthiazole, benzimidazole, benztriazole, thioxanthine, carbazole, carboline, acridine, pyrolizidine, and quinolizidine.

In addition to the polycyclic heterocycles described above, heterocycle includes polycyclic heterocycles wherein the ring fusion between two or more rings includes more than one bond common to both rings and more than two atoms common to both rings. Examples of such bridged heterocycles include quinuclidine, diazabicyclo[2.2.1]heptane and 7-oxabicyclo[2.2.1]heptane.

Heterocyclyl includes, for example, monocyclic heterocyclyls, such as: aziridinyl, oxiranyl, thiiranyl, azetidinyl, oxetanyl, thietanyl, pyrrolidinyl, pyrrolinyl, imidazolidinyl, pyrazolidinyl, pyrazolinyl, dioxolanyl, sulfolanyl, 2,3-dihydrofuranyl, 2,5-dihydrofuranyl, tetrahydrofuranyl, thiophanyl, piperidinyl, 1,2,3,6-tetrahydropyridinyl, piperazinyl, morpholinyl, thiomorpholinyl, pyranyl, thiopyranyl, 2,3-dihydropyranyl, tetrahydropyranyl, 1,4-dihydropyridinyl, 1,4-dioxanyl, 1,3-dioxanyl, dioxanyl, homopiperidinyl, 2,3,4,7-tetrahydro-1*H*-azepinyl, homopiperazinyl, 1,3-dioxepanyl, 4,7-dihydro-1,3-dioxepinyl, and hexamethylene oxidyl.

In addition, heterocyclyl includes aromatic heterocyclyls or heteroaryl, for example, pyridinyl, pyrazinyl, pyrimidinyl, pyridazinyl, thienyl, furyl, furazanyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4 oxadiazolyl.

Additionally, heterocyclyl encompasses polycyclic heterocyclyls (including both aromatic or non-aromatic), for example, indolyl, indolinyl, isoindolinyl, quinolinyl, tetrahydroquinolinyl, isoquinolinyl, tetrahydroisoquinolinyl, 1,4-benzodioxanyl, coumarinyl, dihydrocoumarinyl, benzofuranyl, 2,3-dihydrobenzofuranyl, isobenzofuranyl, chromenyl, chromanyl, isochromanyl, xanthenyl, phenoxathiinyl, thianthrenyl, indolizinyl, isoindolyl, indazolyl, purinyl, phthalazinyl, naphthyridinyl, quinoxalinyl, quinazolinyl, cinnolinyl, pteridinyl, phenanthridinyl, perimidinyl, phenanthrolinyl, phenazinyl, phenothiazinyl, phenoxazinyl, 1,2-benzisoxazolyl, benzothiophenyl, benzoxazolyl, benzthiazolyl, benzimidazolyl, benztriazolyl, thioxanthinyl, carbazolyl, carbolinyl, acridinyl, pyrolizidinyl, and quinolizidinyl.

In addition to the polycyclic heterocyclyls described above, heterocyclyl includes polycyclic heterocyclyls wherein the ring fusion between two or more rings includes more than one bond common to both rings and more than two atoms common to both rings. Examples of such bridged heterocycles include quinuclidinyl, diazabicyclo[2.2.1]heptyl; and 7-oxabicyclo[2.2.1]heptyl.

The term "alkoxy" used alone or as a suffix or prefix, refers to radicals of the general formula -O-R, wherein R is selected from a hydrocarbon radical. Exemplary alkoxy includes methoxy, ethoxy, propoxy, isopropoxy, butoxy, t-butoxy, isobutoxy, cyclopropylmethoxy, allyloxy, and propargyloxy.

The term "amine" or "amino" used alone or as a suffix or prefix, refers to radicals of the general formula -NRR', wherein R and R' are independently selected from hydrogen or a hydrocarbon radical.

"Acyl" used alone, as a prefix or suffix, means -C(=O)-R, wherein R is an optionally substituted hydrocarbyl, hydrogen, amino or alkoxy. Acyl groups include, for example, acetyl, propionyl, benzoyl, phenyl acetyl, carboethoxy, and dimethylcarbamoyl.

Halogen includes fluorine, chlorine, bromine and iodine.

"Halogenated," used as a prefix of a group, means one or more hydrogens on the group is replaced with one or more halogens.

"RT" or "rt" means room temperature.

A first ring group being "fused" with a second ring group means the first ring and the second ring share at least two atoms therebetween.

"Link," "linked," or "linking," unless otherwise specified, means covalently linked or bonded.

Provided herein is a compound of formula I, a pharmaceutically acceptable salt thereof, diastereomers thereof, enantiomers thereof, or mixtures thereof:

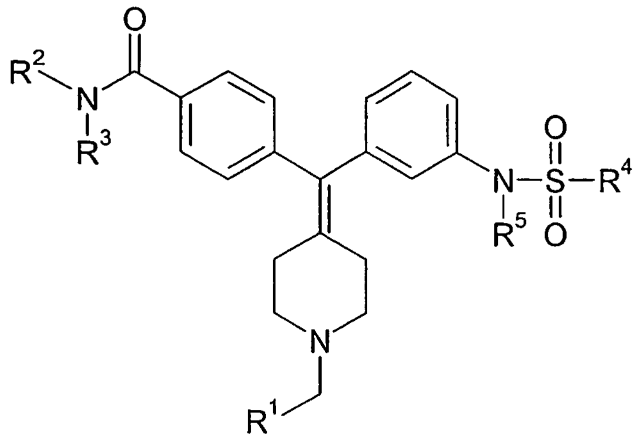

I

wherein

$R^1$ is selected from $C_{6-10}$aryl and $C_{2-6}$heteroaryl, wherein said $C_{6-10}$aryl and $C_{2-6}$heteroaryl are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R, and-NRC(=O)-OR, wherein R is, independently, a hydrogen or $C_{1-6}$alkyl; and

$R^2$, $R^3$, $R^4$ and $R^5$ are, independently, selected from hydrogen, $C_{1-6}$alkyl, and $C_{3-6}$cycloalkyl, wherein said $C_{1-6}$alkyl and $C_{3-6}$cycloalkyl are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH,-C(=O)OR, $-C(=O)NR_2$, -NRC(=O)R, and -NRC(=O)-OR, wherein R is, independently, a hydrogen or $C_{1-6}$alkyl.

One embodiment of the compounds of the invention may be those of formula I, wherein $R^1$ is selected from phenyl; pyridyl; thienyl; furyl; imidazolyl; triazolyl; pyrrolyl; thiazolyl; and N-oxido-pyridyl, wherein $R^1$ is optionally substituted with one or more groups selected from $C_{1-6}$alkyl, halogenated $C_{1-6}$alkyl, $-NO_2$, $-CF_3$, $C_{1-6}$ alkoxy, chloro, fluoro, bromo, and iodo;
$R^2$, $R^3$, and $R^4$ are, independently, $C_{1-3}$alkyl or halogenated $C_{1-3}$alkyl;
$R^5$ is selected from hydrogen, $C_{1-6}$alkyl, and $C_{3-6}$cycloalkyl, wherein said $C_{1-6}$alkyl and $C_{3-6}$cycloalkyl are optionally substituted with one or more groups selected from $C_{1-6}$alkyl, halogenated $C_{1-6}$alkyl, $-NO_2$, $-CF_3$, $C_{1-6}$ alkoxy, chloro, fluoro, bromo, and iodo.

Another embodiment of the compounds of the invention may be those of formula I, wherein $R^1$ is selected from phenyl; pyridyl; thienyl; furyl; imidazolyl; pyrrolyl; and thiazolyl, wherein $R^1$ is optionally substituted with one or more groups selected from $C_{1-6}$alkyl, halogenated $C_{1-6}$alkyl, $-NO_2$, $-CF_3$, $C_{1-6}$ alkoxy, chloro, fluoro, bromo, and iodo;
$R^2$, $R^3$, and $R^4$ are, independently, $C_{1-3}$alkyl or halogenated $C_{1-3}$alkyl; and $R^5$ is hydrogen.

A further embodiment of the compounds of the invention may be those of formula I, wherein $R^1$ is selected from phenyl, pyridyl, thienyl, furyl, imidazolyl, pyrrolyl, and thiazolyl;
$R^2$ and $R^3$ are ethyl;
$R^4$ is $C_{1-3}$alkyl; and
$R^5$ is hydrogen.

It will be understood that when compounds of the present invention contain one or more chiral centers, the compounds of the invention may exist in, and be isolated as, enantiomeric or diastereomeric forms, or as a racemic mixture. The present invention includes any possible enantiomers, diastereomers, racemates or mixtures thereof, of a compound of Formula I. The optically active forms of the compound of the invention may be prepared, for example, by chiral chromatographic separation of a racemate, by synthesis from optically active starting materials or by asymmetric synthesis based on the procedures described thereafter.

It will also be appreciated that certain compounds of the present invention may exist as geometrical isomers, for example E and Z isomers of alkenes. The present invention includes any geometrical isomer of a compound of Formula I. It will further be understood that the present invention encompasses tautomers of the compounds of the formula I.

It will also be understood that certain compounds of the present invention may exist in solvated, for example hydrated, as well as unsolvated forms. It will further be understood that the present invention encompasses all such

solvated forms of the compounds of the formula I.

Within the scope of the invention are also salts of the compounds of the formula I. Generally, pharmaceutically acceptable salts of compounds of the present invention may be obtained using standard procedures well known in the art, for example by reacting a sufficiently basic compound, for example an alkyl amine with a suitable acid, for example, HCl or acetic acid, to afford a physiologically acceptable anion. It may also be possible to make a corresponding alkali metal (such as sodium, potassium, or lithium) or an alkaline earth metal (such as a calcium) salt by treating a compound of the present invention having a suitably acidic proton, such as a carboxylic acid or a phenol with one equivalent of an alkali metal or alkaline earth metal hydroxide or alkoxide (such as the ethoxide or methoxide), or a suitably basic organic amine (such as choline or meglumine) in an aqueous medium, followed by conventional purification techniques.

In one embodiment, the compound of formula I above may be converted to a pharmaceutically acceptable salt or solvate thereof, particularly, an acid addition salt such as a hydrochloride, hydrobromide, phosphate, acetate, fumarate, maleate, tartrate, citrate, methanesulphonate or *p*-toluenesulphonate.

The novel compounds, of the present invention are useful in therapy, especially for the treatment of various pain conditions such as chronic pain, neuropathic pain, acute pain, cancer pain, pain caused by rheumatoid arthritis, migraine, visceral pain etc. This list should however not be interpreted as exhaustive.

Compounds of the invention are useful as immunomodulators, especially for autoimmune diseases, such as arthritis, for skin grafts, organ transplants and similar surgical needs, for collagen diseases, various allergies, for use as anti-tumour agents and anti viral agents.

Compounds of the invention are useful in disease states where degeneration or dysfunction of opioid receptors is present or implicated in that paradigm. This may involve the use of isotopically labelled versions of the compounds of the invention in diagnostic techniques and imaging applications such as positron emission tomography (PET).

Compounds of the invention are useful for the treatment of diarrhoea, depression, anxiety and stress-related disorders such as post-traumatic stress disorders, panic disorder, generalized anxiety disorder, social phobia, and obsessive compulsive disorder, urinary incontinence, premature ejaculation, various mental illnesses, cough, lung oedema, various gastro-intestinal disorders, e.g. constipation, functional gastrointestinal disorders such as Irritable Bowel Syndrome and Functional Dyspepsia, Parkinson's disease and other motor disorders, traumatic brain injury, stroke, cardioprotection following miocardial infarction, spinal injury and drug addiction, including the treatment of alcohol, nicotine, opioid and other drug abuse and for disorders of the sympathetic nervous system for example hypertension.

Compounds of the invention are useful as an analgesic agent for use during general anaesthesia and monitored anaesthesia care. Combinations of agents with different properties are often used to achieve a balance of effects needed to maintain the anaesthetic state (e.g. amnesia, analgesia, muscle relaxation and sedation). Included in this combination are inhaled anaesthetics, hypnotics, anxiolytics, neuromuscular blockers and opioids.

Also within the scope of the invention is the use of any of the compounds according to the formula I above, for the manufacture of a medicament for the treatment of any of the conditions discussed above.

A further aspect of the invention is a method for the treatment of a subject suffering from any of the conditions discussed above, whereby an effective amount of a compound according to the formula I above, is administered to a patient in need of such treatment.

Thus, the invention provides a compound of formula I, or pharmaceutically acceptable salt or solvate thereof, as hereinbefore defined for use in therapy.

In a further aspect, the present invention provides the use of a compound of formula I, or a pharmaceutically acceptable salt or solvate thereof, as hereinbefore defined in the manufacture of a medicament for use in therapy.

In the context of the present specification, the term "therapy" also includes "prophylaxis" unless there are specific indications to the contrary. The term "therapeutic" and "therapeutically" should be contrued accordingly. The term "therapy" within the context of the present invention further encompasses to administer an effective amount of a compound of the present invention, to mitigate either a pre-existing disease state, acute or chronic, or a recurring condition. This definition also encompasses prophylactic therapies for prevention of recurring conditions and continued therapy for chronic disorders.

The compounds of the present invention are useful in therapy, especially for the therapy of various pain conditions including, but not limited to: acute pain, chronic pain, neuropathic pain, acute pain, back pain, cancer pain, and visceral pain.

In use for therapy in a warm-blooded animal such as a human, the compound of the invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints.

In one embodiment of the invention, the route of administration may be orally, intravenously or intramuscularly.

The dosage will depend on the route of administration, the severity of the disease, age and weight of the patient and other factors normally considered by the attending physician, when determining the individual regimen and dosage level at the most appropriate for a particular patient.

For preparing pharmaceutical compositions from the compounds of this invention, inert, pharmaceutically acceptable carriers can be either solid and liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or table disintegrating agents; it can also be an encapsulating material.

In powders, the carrier is a finely divided solid, which is in a mixture with the finely divided compound of the invention, or the active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture in then poured into convenient sized moulds and allowed to cool and solidify.

Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term composition is also intended to include the formulation of the active component with encapsulating material as a carrier providing a capsule in which the active component (with or without other carriers) is surrounded by a carrier which is thus in association with it. Similarly, cachets are included.

Tablets, powders, cachets, and capsules can be used as solid dosage forms suitable for oral administration.

Liquid form compositions include solutions, suspensions, and emulsions. For example, sterile water or water propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Depending on the mode of administration, the pharmaceutical composition will preferably include from 0.05% to 99%w (per cent by weight), more preferably from 0.10 to 50%w, of the compound of the invention, all percentages by weight being based on total composition.

A therapeutically effective amount for the practice of the present invention may be determined, by the use of known criteria including the age, weight and response of the individual patient, and interpreted within the context of the disease which is being treated or which is being prevented, by one of ordinary skills in the art.

Within the scope of the invention is the use of any compound of formula I as defined above for the manufacture of a medicament.

Also within the scope of the invention is the use of any compound of formula I for the manufacture of a medicament for the therapy of pain.

Additionally provided is the use of any compound according to Formula I for the manufacture of a medicament for the therapy of various pain conditions including, but not limited to: acute pain, chronic pain, neuropathic pain, acute pain, back pain, cancer pain, and visceral pain.

A further aspect of the invention is a method for therapy of a subject suffering from any of the conditions discussed above, whereby an effective amount of a compound according to the formula I above, is administered to a patient in need of such therapy.

Additionally, there is provided a pharmaceutical composition comprising a compound of Formula I, or a pharmaceutically acceptable salt thereof, in association with a pharmaceutically acceptable carrier.

Particularly, there is provided a pharmaceutical composition comprising a compound of Formula I, or a pharmaceutically acceptable salt thereof, in association with a pharmaceutically acceptable carrier for therapy, more particularly for therapy of pain.

Further, there is provided a pharmaceutical composition comprising a compound of Formula I, or a pharmaceutically acceptable salt thereof, in association with a pharmaceutically acceptable carrier use in any of the conditions discussed above.

Also provided herein is a method of preparing a compound of formula I.

In one embodiment, the invention provides a process for preparing a compound of formula I, comprising:

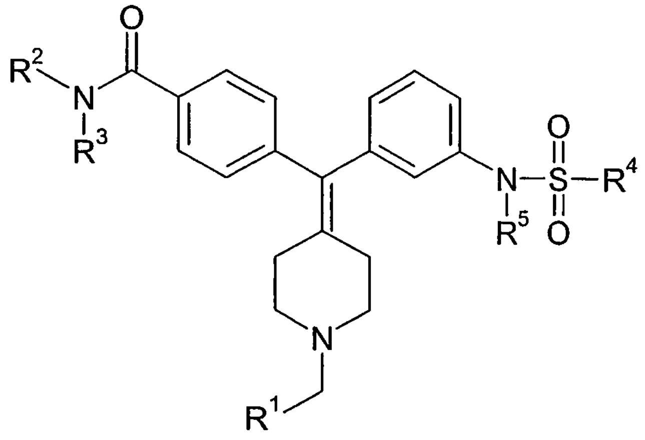

I

reacting a compound of formula II with $X-S(=O)_2-R^4$ or $R^4S(=O)_2-O-S(=O)_2R^4$.

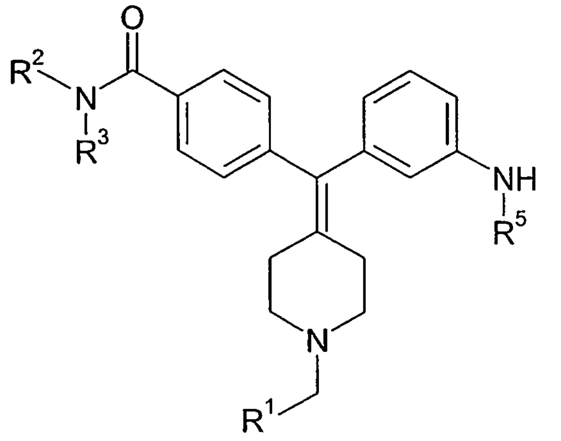

II

wherein

X is selected from Cl, Br and I;
$R^1$ is selected from $C_{6-10}$aryl and $C_{2-6}$heteroaryl, wherein said $C_{6-10}$aryl and $C_{2-6}$heteroaryl are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R, and-NRC(=O)-OR, wherein R is, independently, a hydrogen or $C_{1-6}$alkyl; and
$R^2$, $R^3$, $R^4$ and $R^5$ are, independently, selected from hydrogen, $C_{1-6}$alkyl, and $C_{3-6}$cycloalkyl, wherein said $C_{1-6}$alkyl and $C_{3-6}$cycloalkyl are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH,-C(=O)OR, $-C(=O)NR_2$, -NRC(=O)R, and -NRC(=O)-OR, wherein R is, independently, a hydrogen or $C_{1-6}$alkyl.

Particularly, the compounds of the present invention and intermediates used for the preparation thereof can be prepared according to the synthetic routes as exemplified in Schemes 1, 2 and 3.

Scheme 1

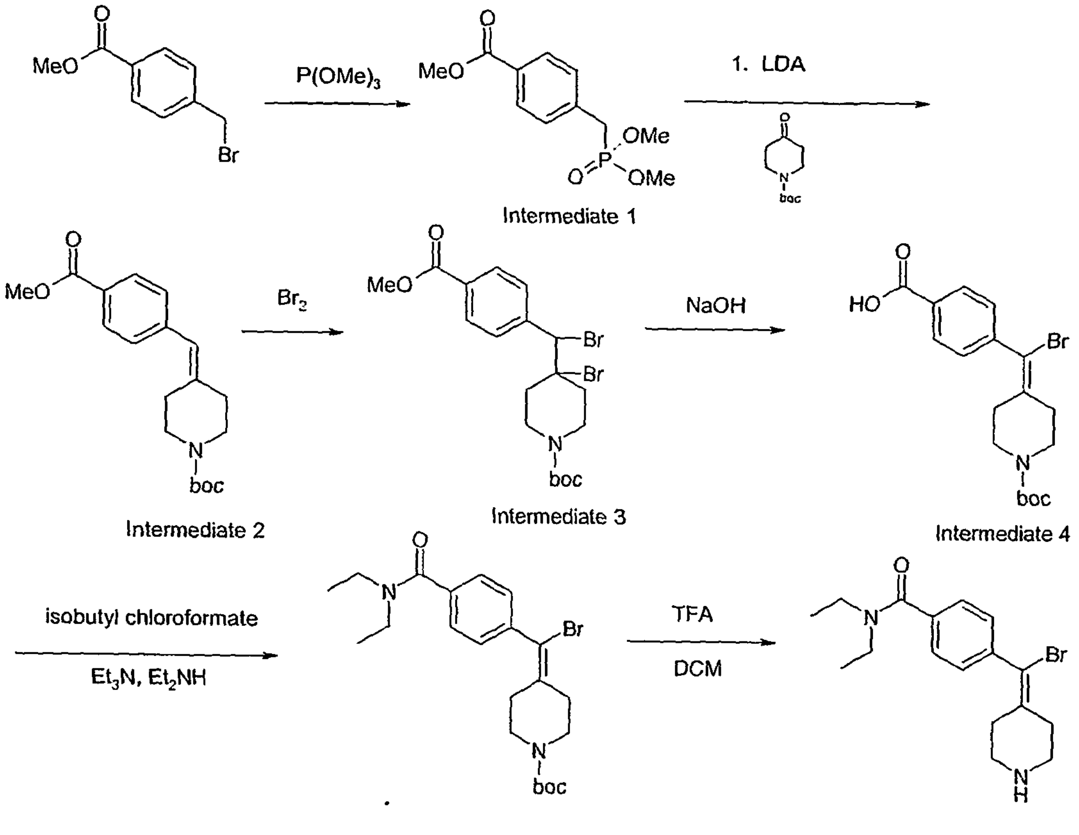


Intermediate 1

Intermediate 2

Intermediate 3

Intermediate 4

Intermediate 5

Intermediate 6

Scheme 2

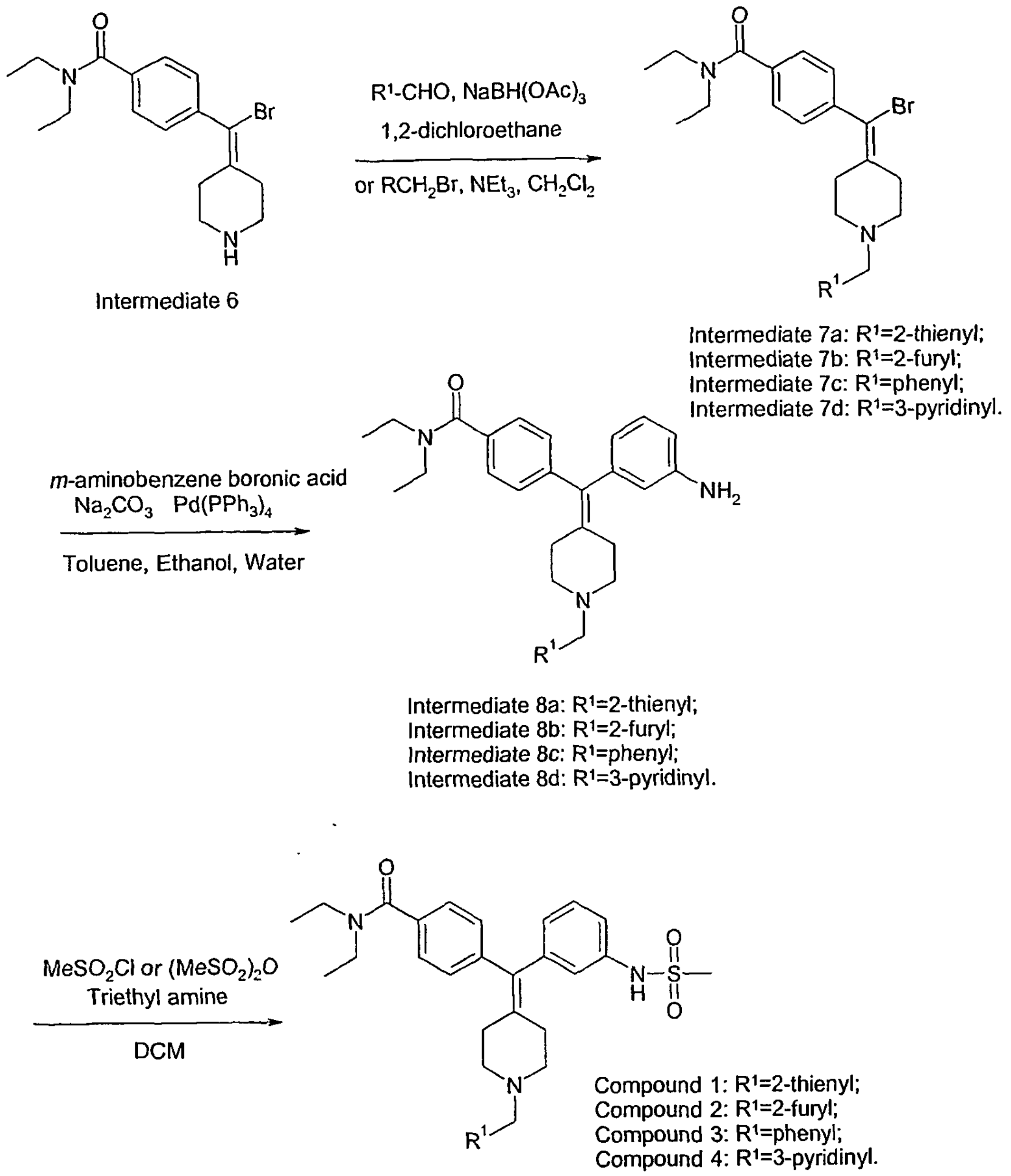


Intermediate 6

Intermediate 7a: $R^1$=2-thienyl;
Intermediate 7b: $R^1$=2-furyl;
Intermediate 7c: $R^1$=phenyl;
Intermediate 7d: $R^1$=3-pyridinyl.

Intermediate 8a: $R^1$=2-thienyl;
Intermediate 8b: $R^1$=2-furyl;
Intermediate 8c: $R^1$=phenyl;
Intermediate 8d: $R^1$=3-pyridinyl.

Compound 1: $R^1$=2-thienyl;
Compound 2: $R^1$=2-furyl;
Compound 3: $R^1$=phenyl;
Compound 4: $R^1$=3-pyridinyl.

Scheme 3

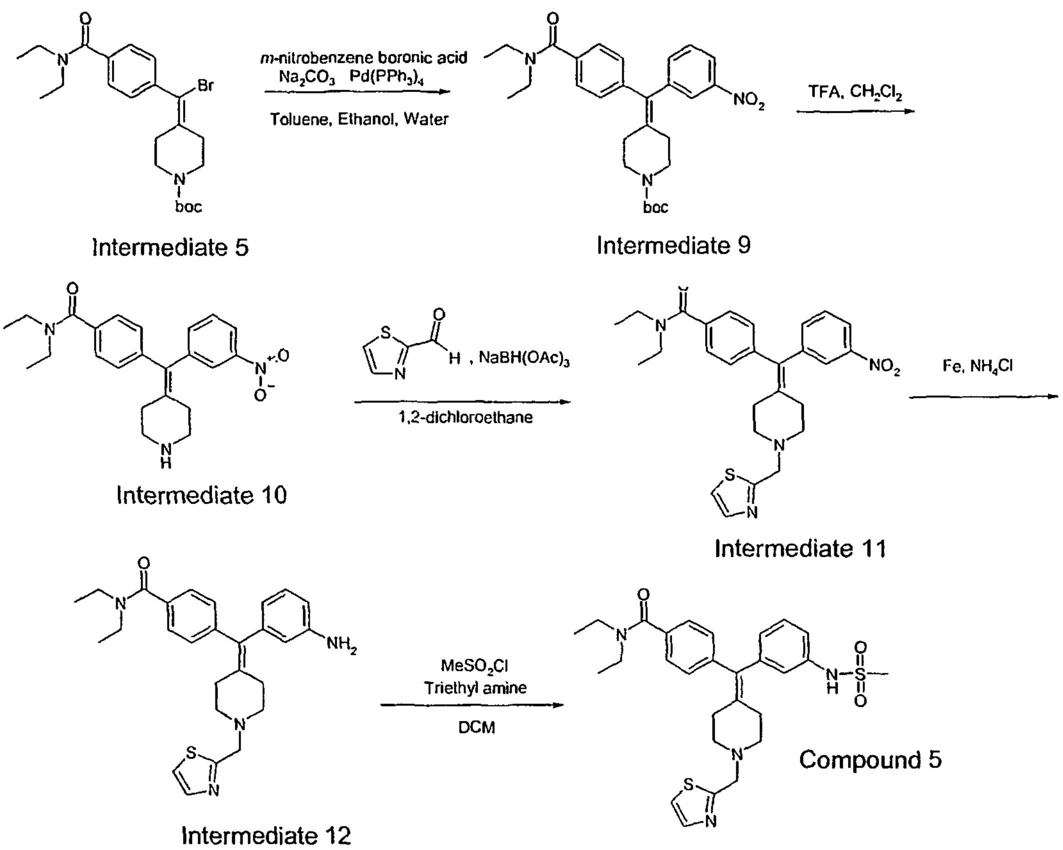


Intermediate 5

Intermediate 9

Intermediate 10

Intermediate 11

Intermediate 12

Compound 5

## BIOLOGICAL EVALUATION

The compounds of the invention are found to be active towards δ receptors in warm-blooded animal, e.g., human. Particularly the compounds of the invention are found to be effective δ receptor ligands. *In vitro* assays, *infra,* demonstrate these surprising activities, especially with regard to agonists potency and efficacy as demonstrated in the rat brain functional assay and/or the human δ receptor functional assay (low). This feature may be related to in vivo activity and may not be linearly correlated with binding affinity. In these *in vitro* assays, a compound is tested for their activity toward δ receptors and $IC_{50}$ is obtained to determine the selective activity for a particular compound towards δ receptors. In the current context, $IC_{50}$ generally refers to the concentration of the compound at winch 50% displacement of a standard radioactive δ receptor ligand has been observed.

The activities of the compound towards κ and μ receptors are also measured in a similar assay.

In vitro model

Cell culture

Human 2935 cells expressing cloned human κ, δ and μ receptors and neomycin resistance are grown in suspension at 37°C and 5% $CO_2$ in shaker flasks containing calcium-free DMEM10% FBS, 5% BCS, 0.1% Pluronic F-68, and 600 μg/ml geneticin.

Rat brains are weighed and rinsed in ice-cold PBS (containing 2.5mM EDTA, pH 7.4). The brains are homogenized with a polytron for 30 sec (rat) in ice-cold lysis buffer (50mM Tris, pH 7.0, 2.5mM EDTA, with phenylmethylsulfonyl fluoride added just prior use to 0.5MmM from a 0.5M stock in DMSO:ethanol).

Membrane preparation

Cells are pelleted and resuspended in lysis buffer (50 mM Tris, pH 7.0, 2.5 mM EDTA, with PMSF added just

prior to use to 0.1 mM from a 0.1 M stock in ethanol), incubated on ice for 15 min, then homogenized with a polytron for 30 sec. The suspension is spun at 1000g (max) for 10 min at 4°C. The supernatant is saved on ice and the pellets resuspended and spun as before. The supernatants from both spins are combined and spun at 46,000 g(max) for 30 min. The pellets are resuspended in cold Tris buffer (50 mM Tris/Cl, pH 7.0) and spun again. The final pellets are resuspended in membrane buffer (50 mM Tris, 0.32 M sucrose, pH 7.0). Aliquots (1 ml) in polypropylene tubes are frozen in dry ice/ethanol and stored at -70°C until use. The protein concentrations are determined by a modified Lowry assay with sodium dodecyl sulfate.

Binding assays

Membranes are thawed at 37°C, cooled on ice, (or kept on ice if not used immediately) passed 3 times through a 25-gauge needle, and diluted into binding buffer (50 mM Tris, 3 mM $MgCl_2$, 1 mg/ml BSA (Sigma A-7888), pH 7.4, which is stored at 4°C after filtration through a 0.22 m filter, and to which has been freshly added 5 μg/ml aprotinin, 10 μM bestatin, 10 μM diprotin A if the membranes are derived from tissue (rat, mouse, monkey, no DTT). Aliquots of 100 μl are added to iced 12x75 mm polypropylene tubes containing 100 μl of the appropriate radioligand and 100 μl of test compound at various concentrations. Total (TB) and nonspecific (NS) binding are determined in the absence and presence of 10 μM naloxone respectively. The tubes are vortexed and incubated at 25°C for 60-75 min, after which time the contents are rapidly vacuum-filtered and washed with about 12 ml/tube iced wash buffer (50 mM Tris, pH 7.0, 3 mM $MgCl_2$) through GF/B filters (Whatman) presoaked for at least 2h in 0.1% polyethyleneimine. The radioactivity (dpm) retained on the filters is measured with a beta counter after soaking the filters for at least 12h in minivials containing 6-7 ml scintillation fluid. If the assay is set up in 96-place deep well plates, the filtration is over 96-place PEI-soaked unifilters, which are washed with 3 x 1 ml wash buffer, and dried in an oven at 55°C for 2h. The filter plates are counted in a TopCount (Packard) after adding 50 μl MS-20 scintillation fluid/well. In the case of assays performed in 96 deep well plates, the IC50 of compounds are evaluated from 10-point displacement curves in the case of Delta, and 5-point displacement curves in the case of Mu and Kappa. The assay is done in 300μl with the appropriate amount of membrane protein (2μg, 35μg, and 1μg, in the case of Delta, Mu, and Kappa, respectively) and 50000-80000 dpm/well of the appropriate tracer (125I-Deltorphin II, 125I-FK33824, and 125I-DPDYN for Delta, Mu, and Kappa, respectively). The total binding and non-specific binding are determined in absence and presence of 10μM of Naloxone.

Functional Assays

The agonist activity of the compounds is measured by determining the degree to which the compounds receptor complex activates the binding of GTP to G-proteins to which the receptors are coupled. In the GTP binding assay, GTP $[\gamma]^{35}S$ is combined with test compounds and membranes from HEK-293S cells expressing the cloned human opioid receptors or from homogenised rat or mouse brain. Agonists stimulate GTP$[\gamma]^{35}S$ binding in these membranes. The $EC_{50}$ and $E_{max}$ values of compounds are determined from dose-response curves. Right shifts of the dose response curve by the delta antagonist naltrindole are performed to verify that agonist activity is mediated through delta receptors. For human δ receptor functional assays, $EC_{50}$ (low) is measured when the human δ receptors used in the assay were expressed at lower levels in comparison with those used in determining $EC_{50}$ (high). The $E_{max}$ values were determined in relation to the standard δ agonist SNC80, i.e., higher than 100% is a compound that have better efficacy than SNC80.

Procedure for rat brain GTP

Rat brain membranes are thawed at 37°C, passed 3 times through a 25-gauge blunt-end needle and diluted in the GTPγS binding (50 mM Hepes, 20 mM NaOH, 100 mM NaCl, 1 mM EDTA, 5 mM $MgCl_2$, pH 7.4, Add fresh: 1 mM DTT, 0.1% BSA). 120μM GDP final is added membranes dilutions. The EC50 and Emax of compounds are evaluated from 10-point dose-response curves done in 300μl with the appropriate amount of membrane protein (20μg/well) and 100000-130000 dpm of GTP$\gamma^{35}S$ per well (0.11 -0.14nM). The basal and maximal stimulated binding are determined in absence and presence of 3 μM SNC-80. The assay performed on HEK 293s cells stably expressing cloned Delta receptors is done in a slightly different buffer (50mM Hepes, 20mM NaOH, 200mM NaCl, 1 mM EDTA, 5mM $MgCl_2$, pH 7.4, Add fresh: 0.5% BSA, no DTT) and with a 3μM final conc. of GDP.

Data analysis

The specific binding (SB) was calculated as TB-NS, and the SB in the presence of various test compounds was expressed as percentage of control SB. Values of $IC_{50}$ and Hill coefficient ($n_H$) for ligands in displacing specifically bound radioligand were calculated from logit plots or curve fitting programs such as Ligand, GraphPad Prism, SigmaPlot, or ReceptorFit. Values of $K_i$ were calculated from the Cheng-Prussoff equation. Mean ± S.E.M. values of $IC_{50}$, $K_i$ and

$n_H$ were reported for ligands tested in at least three displacement curves. Biological activity of the compounds of the present invention is indicated in Tables 1 and 2.

Table 1

| Compd. # | Human δ (nM) | | | Human κ (nM) | Human μ (nM) | RAT BRAIN (nM) | |
|---|---|---|---|---|---|---|---|
| | $IC_{50}$ | $EC_{50}$ (high) | %EMax (high) | $IC_{50}$ | $IC_{50}$ | $EC_{50}$ | %EMax |
| 3 | 0.56 | 0.53 | 99 | 1046 | 35 | 1.32 | 145 |

Table 2

| Compd. # | Human δ (nM) | | | Human κ (nM) | Human μ (nM) |
|---|---|---|---|---|---|
| | $IC_{50}$ | $EC_{50}$ (low) | %EMax (low) | $IC_{50}$ | $IC_{50}$ |
| 1,2,4,5 | 0.18-0.35 | 5.25-19.33 | 106.5-126.4 | 700.1-3891.1 | 77.9-300.5 |

Receptor Saturation Experiments

Radioligand $K_\delta$ values are determined by performing the binding assays on cell membranes with the appropriate radioligands at concentrations ranging from 0.2 to 5 times the estimated $K_\delta$ (up to 10 times if amounts of radioligand required are feasible). The specific radioligand binding is expressed as pmole/mg membrane protein. Values of $K_\delta$ and $B_{max}$ from individual experiments are obtained from nonlinear fits of specifically bound (B) vs. nM free (F) radioligand from individual according to a one-site model.

Determination Of Mechano-Allodynia Using Von Frey Testing

Testing is performed between 08:00 and 16:00h using the method described by Chaplan et al. (1994). Rats are placed in Plexiglas cages on top of a wire mesh bottom which allows access to the paw, and are left to habituate for 10-15 min. The area tested is the mid-plantar left hind paw, avoiding the less sensitive foot pads. The paw is touched with a series of 8 Von Frey hairs with logarithmically incremental stiffness (0.41, 0.69, 1.20, 2.04, 3.63, 5.50, 8.51, and 15.14 grams; Stoelting, Ill, USA). The von Frey hair is applied from underneath the mesh floor perpendicular to the plantar surface with sufficient force to cause a slight buckling against the paw, and held for approximately 6-8 seconds. A positive response is noted if the paw is sharply withdrawn. Flinching immediately upon removal of the hair is also considered a positive response. Ambulation is considered an ambiguous response, and in such cases the stimulus is repeated.

Testing Protocol

The animals are tested on postoperative day 1 for the FCA-treated group. The 50% withdrawal threshold is determined using the up-down method of Dixon (1980). Testing is started with the 2.04 g hair, in the middle of the series. Stimuli are always presented in a consecutive way, whether ascending or descending. In the absence of a paw withdrawal response to the initially selected hair, a stronger stimulus is presented; in the event of paw withdrawal, the next weaker stimulus is chosen. Optimal threshold calculation by this method requires 6 responses in the immediate vicinity of the 50% threshold, and counting of these 6 responses begins when the first change in response occurs, e.g. the threshold is first crossed. In cases where thresholds fall outside the range of stimuli, values of 15.14 (normal sensitivity) or 0.41 (maximally allodynic) are respectively assigned. The resulting pattern of positive and negative responses is tabulated using the convention, X = no withdrawal; O = withdrawal, and the 50% withdrawal threshold is interpolated using the formula:

$$50\%\ g\ threshold = 10^{(Xf + k\delta)} / 10{,}000$$

where Xf= value of the last von Frey hair used (log units); k = tabular value (from Chaplan et al. (1994)) for the pattern of positive / negative responses; and δ = mean difference between stimuli (log units). Here δ = 0.224.

Von Frey thresholds are converted to percent of maximum possible effect (% MPE), according to Chaplan et al. 1994. The following equation is used to compute % MPE:

$$\% \text{ MPE} = \frac{\text{Drug treated threshold (g) - allodynia threshold (g)}}{\text{Control threshold (g) - allodynia threshold (g)}} \times 100$$

Administration Of Test Substance

Rats are injected (subcutaneously, intraperitoneally, intravenously or orally) with a test substance prior to von Frey testing, the time between administration of test compound and the von Frey test varies depending upon the nature of the test compound.

Writhing Test

Acetic acid will bring abdominal contractions when administered intraperitoneally in mice. These will then extend their body in a typical pattern. When analgesic drugs are administered, this described movement is less frequently observed and the drug selected as a potential good candidate.

A complete and typical Writhing reflex is considered only when the following elements are present: the animal is not in movement; the lower back is slightly depressed; the plantar aspect of *both* paws is observable. In this assay, compounds of the present invention demonstrate significant inhibition of writhing responses after oral dosing of 1-100 μmol/kg.

(i) Solutions preparation

Acetic acid (AcOH): 120 μL of Acetic Acid is added to 19.88 ml of distilled water in order to obtain a final volume of 20 ml with a final concentration of 0.6% AcOH. The solution is then mixed (vortex) and ready for injection.

Compound (drug): Each compound is prepared and dissolved in the most suitable vehicle according to standard procedures.

(ii) Solutions administration

The compound (drug) is administered orally, intraperitoneally (i.p.), subcutaneously (s.c.) or intravenously (i.v.)) at 10 ml/kg (considering the average mice body weight) 20, 30 or 40 minutes (according to the class of compound and its characteristics) prior to testing. When the compound is delivered centrally: Intraventricularly (i.c.v.) or intrathecally (i.t.) a volume of 5 μL is administered.

The AcOH is administered intraperitoneally (i.p.) in two sites at 10 ml/kg (considering the average mice body weight) immediately prior to testing.

(iii) Testing

The animal (mouse) is observed for a period of 20 minutes and the number of occasions (Writhing reflex) noted and compiled at the end of the experiment. Mice are kept in individual "shoe box" cages with contact bedding. A total of 4 mice are usually observed at the same time: one control and three doses of drug.

For the anxiety and anxiety-like indications, efficacy has been established in the geller-seifter conflict test in the rat.

For the functional gastrointestina disorder indication, efficacy can be established in the assay described by Coutinho SV *et al,* in American Journal of Physiology - Gastrointestinal & Liver Physiology. 282(2):G307-16, 2002 Feb, in the rat.

## ADDITIONAL IN VIVO TESTING PROTOCOLS

### Subjects and housing

Naive male Sprague Dawley rats (175-200g) are housed in groups of 5 in a temperature controlled room (22°C, 40-70% humidity, 12-h light/dark). Experiments are performed during the light phase of the cycle. Animals have food and water ad libitum and are sacrificed immediately after data acquisition.

### Sample

Compound (Drug) testing includes groups of rats that do not receive any treatment and others that are treated with E. coli lipopolysaccharide(LPS). For the LPS-treated experiment, four groups are injected with LPS, one of the four groups is then vehicle-treated whilst the other three groups are injected with the drug and its vehicle. A second set of experiments are conducted involving five groups of rats; all of which receive no LPS treatment. The naïve group receives no compound (drug) or vehicle; the other four groups are treated with vehicle with or without drug. These are performed to determine anxiolytic or sedative effects of drugs which can contribute to a reduction in USV.

### Administration of LPS

Rats are allowed to habituate in the experimental laboratory for 15-20 min prior to treatment. Inflammation is induced by administration of LPS (endotoxin of gram-negative E. coli bacteria serotype 0111:B4, Sigma). LPS (2.4μg) is injected intracerebro-ventricularly (i.c.v.), in a volume of 10μl, using standard stereotaxic surgical techniques under isoflurane anaesthesia. The skin between the ears is pushed rostrally and a longitudinal incision of about 1cm is made to expose the skull surface. The puncture site is determined by the coordinates: 0.8 mm posterior to the bregma, 1.5 mm lateral (left) to the lambda (sagittal suture), and 5 mm below the surface of the skull (vertical) in the lateral ventricle. LPS is injected via a sterile stainless steel needle (26-G 3/8) of 5 mm long attached to a 100-μl Hamilton syringe by polyethylene tubing (PE20; 10-15 cm). A 4 mm stopper made from a cut needle (20-G) is placed over and secured to the 26-G needle by silicone glue to create the desired 5mm depth.

Following the injection of LPS, the needle remains in place for an additional 10 s to allow diffusion of the compound, then is removed. The incision is closed, and the rat is returned to its original cage and allowed to rest for a minimum of 3.5h prior to testing.

### Experimental setup for air-puff stimulation

The rats remains in the experimental laboratory following LPS injection and compound (drug) administration. At the time of testing all rats are removed and placed outside the laboratory. One rat at a time is brought into the testing laboratory and placed in a clear box (9 × 9 × 18 cm) which is then placed in a sound-attenuating ventilated cubicle measuring 62(w) ×35(d) ×46(h) cm (BRS/LVE, Div. Tech-Serv Inc). The delivery of air-puffs, through an air output nozzle of 0.32 cm, is controlled by a system (AirStim, San Diego Intruments) capable of delivering puffs of air of fixed duration (0.2 s) and fixed intensity with a frequency of 1 puff per 10s. A maximun of 10 puffs are administered, or until vocalisation starts, which ever comes first. The first air puff marks the start of recording.

### Experimental setup for and ultrasound recording

The vocalisations are recorded for 10 minutes using microphones (G.R.A.S. sound and vibrations, Vedbaek, Denmark) placed inside each cubicle and controlled by LMS (LMS CADA-X 3.5B, Data Acquisition Monitor, Troy, Michigan) software. The frequencies between 0 and 32000Hz are recorded, saved and analysed by the same software (LMS CADA-X 3.5B, Time Data Processing Monitor and UPA (User Programming and Analysis)).

### Compounds (Drugs)

All compounds (drugs) are pH-adjusted between 6.5 and 7.5 and administered at a volume of 4 ml/kg. Following compound (drug) administration, animals are returned to their original cages until time of testing.

### Analysis

The recording is run through a series of statistical and Fourier analyses to filter (between 20-24kHz) and to calculate the parameters of interest. The data are expressed as the mean ± SEM. Statistical significance is assessed

using T-test for comparison between naive and LPS-treated rats, and one way ANOVA followed by Dunnett's multiple comparison test (post-hoc) for drug effectiveness. A difference between groups is considered significant with a minimum p value of ≤0.05. Experiments are repeated a minimum of two times.

Determination of thermal hyperalgesia using the Hargreaves plantar test

Administration of FCA or carrageen

Freund's Complete Adjuvant (FCA): SIGMA cat.# F 5881, *Mycabacterium tuberculosis* (H37Ra, ATCC 25177), 1mg/ml, heat killed, dried, 0.85 ml paraffin, 0.15 ml mannide monooleate. Or carrageenan Lambda type IV(Cg): SIGMA cat.# C-3889, (Gelatin, vegetable; Irish moss), (1.0% solution) in NaCl.

Injections are done with a Hamilton syringe with a sterile needle size 26G5/8". Rats are handled and placed in chamber for anaesthesia with isoflurane. When the desired effect is reached, the rat is removed and placed on ventral decubitus (sternal position). The left hind paw is grasped and the needle is introduced subcutaneous, ventral aspect, between footpad of finger # 2 and # 3 in order the reach the middle of the paw (metatarsal area). Finally, a volume of 100μl FCA, or 100μl of carrageenan solution, is slowly injected into the paw, and a small pressure is applied for 3-4 seconds after removal of needles.

If the animals are waking up during the procedure, they are then return in the inhalation chamber until desired effect is reached. After the intraplantar injection, the animals are allowed to wake up under observation in their cage.

For FCA treatment, rats are allowed 48 hours for the development of the inflammatory process. For carrageenan treatment, rats are allowed 3 hours for the development of the inflammatory process.On the morning of the test, rats are placed in the lab (in their cages). They are allowed to habituate to the room for at least 30 minutes.

Test Site

The heat stimulus is applied to the center of the plantar surface, in between the pads. The test site must be in contact with the glass, with no urine or feces in between, in order to maintain the correct heat transfer properties from the glass to the skin.

The plantar apparatus consists of a box with a glass top/platform, the glass surface is maintained at 30°C by an internal feedback mechanism. Underneath this glass platform is a light bulb mounted on a moveable arm, a mirror is placed underneath to allow the light to be positioned under the rat's paw. When the light is activated it shines through an aperture of ~2mm diameter. The experimenter activates the light, and automatic sensors turn the light off when the paw is removed; a cut-off of 20.48 seconds ensures that no tissue damage will occur should the rat fail to remove his paw. The experimenter may also turn off the light at any point. A Timer will record the duration of time that the light is activated.

Flux meter: measures the flux/cm2 when the light is activated. This should be maintained at ~97-98; the flux can be modified by adjusting the plantar device, but must never be changed in the middle of an experiment.

Time-Course

The experiment can be performed after varying lengths of time following the induction of inflammation. Hyperalgesia is measured at 48h post-FCA injection or 3h post-carrageenan injection.

Test Procedure

Naive rats: For the procedure of establishing a Dose Response Curve, one group of 7 rats is used as a control group; they are anesthetised with the remaining 28 rats, but are not given any injection. Testing of the naive group may be done either prior to beginning or immediately following the experiment, with the minimum stress possible, the rats are placed in individual Plexiglas boxes (14 x 21 x 9cm) on top of the plantar device; they are allowed to habituate for a period of 30 minutes. When the animals are ready to test, the light is placed directly under the test-site and activated, and the latency to withdrawal is recorded. After a period of 5-8 minutes, to allow skin temperature to return to normal, a second reading is taken, and the rats are then removed and replaced in their cage.

Baseline Values: The remaining 28 rats (divided into 4 groups) that have been injected with FCA (or carrageenan) are placed in individual boxes on the machine and allowed to habituate for 30 minutes. The experimenter should verify the degree of inflammation of the paw and check for discoloration. The heat stimulus is placed under the test site, and the latency to withdrawal is recorded; two readings are taken, as above. It is the comparison of these

baseline values with those of the naive animals that establishes whether hyperalgesia is present.

Post-drug testing: Once hyperalgesia is established, the rats are injected with the compound of interest. Each compound is prepared and dissolved in the most suitable vehicle according to standard procedures. The administration route, doses, volume, and time of testing after injection is specific for that compound (or class of compounds). When testing compounds at 20-30 minutes post-injection, such as for i.v. or s.c. injections, rats are placed and allowed to habituate on the plantar apparatus while the drug produces its effect. When testing compounds at 60 minutes or more following the injection, rats are placed back in their original cage with their cage mates. Rats are always replaced in their original cages with their original cage mates to minimize the stress of re-establishing a social structure within a group of rats. 30min later rats are placed one the plantar and allowed 30 minutes to habituate to the plantar machine. Testing is performed as described above. Two readings are taken Criteria for Testing:

The animal must be calm and quiet, yet alert, and in the correct position, with no urine or feces between the skin of the paw and the glass surface of the machine. An animal should not be tested if:

- The animal is in locomotion, including sniffing, grooming and exploring.
- The animal is sleeping.
- The animal is showing obvious signs of stress (tonic immobility, vocalizations, ears flat), unless these are the possible result of a compound side effect and cannot be avoided.
- The animal is positioned in such a way that the paw is not in direct contact with the glass (paw resting on top of tail);
- The animal's paw is displaying blue coloring as a result of a bad injection. In this case, the animal is rejected from the experiment completely (at the beginning).

When urine or feces are present, the animal is removed, the glass surface is wiped clean, and then the animal is replaced. When the animal is sleeping, or exhibiting tonic immobility, the experimenter may gently move the box or move their hand in front of the box to elicit a short-term attentional behaviour. Close observation of the animal's behaviour should be conducted throughout the test.

Re-Tests:

At any time during the experiment, if the experimenter is not certain that the paw withdrawal response was not a response to the heat stimulus, the animal may be re-tested after 5-8 minutes. This may be due to the animal moving suddenly, or urinating or defecating while the stimulus is being applied.

Acceptable responses:

any of the following are considered responses to the heat stimulus

- Withdrawal movement of the paw off the glass (often followed by paw licking)

    - Lateral movement of the body (contralateral for the stimulated paw)
    - Toes are moving off the glass

- the centroplanar (middle paw) aspect of the inflamed paw is removed from the glass.

Analysis

The data are expressed as the mean ± SEM. Statistical significance is assessed using T-test for comparison between naive and inflamed rats, and one way ANOVA followed by Dunnett's multiple comparison test (post-hoc) for drug effectiveness. A difference between groups is considered significant with a minimum p value of $\leq 0.05$.

**EXAMPLES**

The invention will further be described in more detail by the following Examples which describe methods whereby compounds of the present invention may be prepared, purified, analyzed and biologically tested, and which are not to be construed as limiting the invention.

INTERMEDIATE 1: 4-[(dimethoxyphosphinyl)methyl]-benzoic acid, methyl ester

A mixture of 4-(bromomethyl)benzoic acid, methyl ester (11.2 g, 49 mmol) and trimethyl phosphite (25 mL) was refluxed under $N_2$ for 5 hrs. Excess trimethyl phosphite was removed by co-distillation with toluene to give INTERMEDIATE 1 in quantitative yield. $^1H$ NMR ($CDCl_3$) δ 3.20 (d, 2H, J=22 Hz, $CH_2$), 3.68 (d, 3H 10.8 Hz, $OCH_3$), 3.78 (d, 3H, 11.2 Hz, $OCH_3$), 3.91 (s, 3H, $OCH_3$), 7.38 (m, 2H, Ar-H), 8.00 (d, 2H, J=8 Hz, Ar-H).

INTERMEDIATE 2: 4-(4-Methoxycarbonyl-benzylidene)-piperidine-1-carboxylic acid *tert*-butyl ester

To a solution of INTERMEDIATE 1 in dry THF (200 mL) was added dropwise lithium diisopropylamide (32.7 mL 1.5 M in hexanes, 49 mmol) at -78 °C. The reaction mixture was then allowed to warm to room temperature prior to addition of *N*-*tert*-butoxycarbonyl-4-piperidone (9.76 g, 49 mmol in 100 mL dry THF). After 12 hrs, the reaction mixture was quenched with water (300 mL) and extracted with ethyl acetate (3 x 300 mL). The combined organic phases were dried over $MgSO_4$ and evaporated to give a product, which was purified by flash chromatography to provide INTERMEDIATE 2 as a white solid (5.64 g, 35%). IR (NaCl) 3424, 2974, 2855, 1718, 1 688, 1606, 1427, 1362, 1276 $cm^{-1}$; $^1H$ NMR ($CDCl_3$) δ 1.44 (s, 9H), 2.31 (t, J=5.5 Hz, 2H), 2.42 (t, J=5.5 Hz, 2H), 3.37 (t, J=5.5 Hz, 2H), 3.48 (t, J=5.5 Hz, 2H), 3.87 (s, 3H, $OCH_3$), 6.33 (s, 1H, CH), 7.20 (d J=6.7 Hz, 2H, Ar-H), 7.94 (d, J,=6.7 Hz, 2H, Ar-H); $^{13}C$ NMR ($CDCl_3$) δ 28.3, 29.2, 36.19, 51.9, 123.7, 127.8, 128.7, 129.4, 140.5, 142.1, 154.6, 166.8.

INTERMEDIATE 3: 4-Bromo-4-[bromo-(4-methoxycarbonyl-phenyl)-methyl]-piperidine-1-carboxylic acid tert-butyl ester

To a mixture of INTERMEDIATE 2 (5.2 g, 16 mmol) and $K_2CO_3$ (1.0 g) in dry dichloromethane (200 mL) was added a solution of bromine (2.9 g, 18 mmol) in 30 mL $CH_2Cl_2$ at 0 °C. after 1.5 hrs at room temperature, the solution after filtration of $K_2CO_3$ was condensed. The residue was then dissolved in ethyl acetate (200 mL), washed with water (200 mL), 0.5 M HCl (200 mL) and brine (200 mL), and dried over $MgSO_4$. Removal of solvents provided a product, which was recrystallized from methanol to give INTERMEDIATE 3 as a white solid (6.07 g, 78%). IR (NaCl) 3425, 2969, 1725, 1669, 1426, 1365, 1279, 1243 $cm^{-1}$; $^1H$ NMR ($CDCl_3$) δ 1.28 (s, 9H), 1.75 (m, 1H), 1.90 (m, 1H), 2.1 (m, 2H), 3.08 (br, 2H), 3.90 (s, 3H, $OCH_3$), 4.08 (br, 3H), 7.57 (d, J=8.4 Hz, 2H, Ar-H) 7.98 (d, J=8.4 Hz, 2H, Ar-H); $^{13}C$ NMR ($CDCl_3$) δ 28.3, 36.6, 38.3, 40.3, 52.1, 63.2, 72.9, 129.0, 130.3, 130.4, 141.9, 154.4, 166.3.

INTERMEDIATE 4: 4-[bromo-(4-carboxy-phenyl)-methylene]-piperidine-1-carboxylic acid tert-butyl ester

A solution of INTERMEDIATE 3 (5.4 g 11 mmol) in methanol (300 mL) and 2.0 M NaOH (100 mL) was heated at 40 °C for 3 hrs. The solid was collected by filtration, and dried overnight under vacuum. The dry salt was dissolved in 40% acetonitrile/water, and was adjusted to pH 2 using concentrated HCl. INTERMEDIATE 4 (3.8 g, 87%) was isolated as a white powder by filtration: $^1H$ NMR ($CDCl_3$) δ 1.45 (s, 9H, $^tBu$), 2.22 (dd, J=5.5 Hz, 6.1 Hz, 2H), 2.64 (dd, J=5.5 Hz, 6.1 Hz, 2H), 3.34 (dd, J=5.5 Hz, 6.1 Hz, 2H), 3.54 (dd, J=5.5 Hz, 6.1 Hz, 2H), 7.35 (d, J=6.7 Hz, 2H, Ar-H), 8.08 (d, J=6.7 Hz, 2H, Ar-H); $^{13}C$ NMR ($CDCl_3$) δ 28.3, 31.5, 34.2, 44.0, 115.3, 128.7, 129.4, 130.2, 137.7, 145.2, 154.6, 170.3;.

INTERMEDIATE 5: 4-[bromo-(4-diethylcarbamoyl-phenyl)-methylene]-piperidine-1-carboxylic acid tert-butyl ester

To a solution of INTERMEDIATE 4 (1.0 g, 2.5 mmol) in dry dichloromethane (10 mL) at - 20 °C was added isobutylchloroformate (450 mg, 3.3 mmol). After 20 min at -20 °C diethylamine (4 mL) was added and the reaction was allowed to warm to room temperature. After 1.5 hrs the solvents were evaporated and the residue was partitioned between ethyl acetate and water. The organic phase was washed with brine and dried over $MgSO_4$. Removal of solvents provided a product, which was purified by flash chromatography to give INTERMEDIATE 5 as white needles (800 mg, 73%): IR (NaCl) 3051, 2975, 1694, 1633, 1416, 1281, 1168, 1115 $cm^{-1}$; $^1H$ NMR ($CDCl_3$) δ 1.13 (br, 3H, $CH_3$), 1.22 (br, 3H, $CH_3$), 1.44 (s, 9H, $^tBu$), 2.22 (t, J=5.5 Hz, 2H), 2.62 (t, J=5.5 Hz, 2H), 3.33 (m, 4H), 3.55 (m, 4H), 7.31 (d, J=8.0 Hz, 2H, Ar-H), 7.36 (d, J=8.0 Hz, 2H, Ar-H); $^{13}C$ NMR ($CDCl_3$) δ 12.71, 14.13, 28.3, 31.5, 34.2, 39.1, 43.2, 79.7, 115.9, 126.3, 129.3, 136.8, 137.1, 140.6, 154.6, 170.5.

INTERMEDIATE 6: 4-[bromo(piperidin-4-ylidene)methyl]-*N,N*-diethylbenzamide

To a solution of INTERMEDIATE 5 (15.6 g, 34.6 mmol) in dichloromethane (200 ml) was added trifluoroacetic acid (30 ml, 311 mmol). The solution was stirred 16 hours at room temperature. The solution was then neutralized with saturated $NaHCO_3$ and the aqueous layer was extracted with dichloromethane (3 x 100 mL) and the combined organic extracts were dried ($Na_2SO4$), filtered and concentrated to give INTERMEDIATE 6 as a pale yellow solid (12.05g, 99%).

INTERMEDIATE 7a: 4- {bromo[1-(thien-2-ylmethyl)piperidin-4-ylidene]methyl}-*N,N*-diethylbenzamide

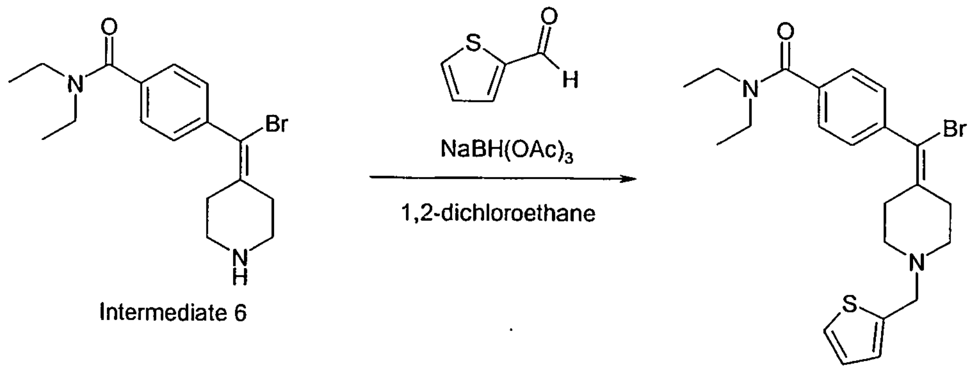


Intermediate 6

Intermediate 7a

To a solution of INTERMEDIATE 6 (1.4g, 3.99mmol) in 1,2-dichloroethane (30ml) was added 2-thiophene carboxaldehyde (746µl, 7.99mmol) and sodium triacetoxyborohydride (1.694g, 7.99mmol). The reaction was stirred at room temperature under nitrogen. After 18 hours the reaction was diluted with dichloromethane and washed with saturated aqueous sodium bicarbonate. The aqueous layer was extracted with two portions of dichloromethane and the combined organic extracts were dried over anhydrous sodium sulfate, filtered and concentrated. The resulting material was purified by flash chromatography, eluting ethyl acetate/hexanes (7:3) to afford INTERMEDIATE 7a (1.702g, 95%) as a thick colourless oil.

INTERMEDIATE 8a: 4-{(3-aminophenyl)[1-(thien-2-ylmethyl)piperidin-4-ylidene]methyl}-*N,N*-diethylbenzamide

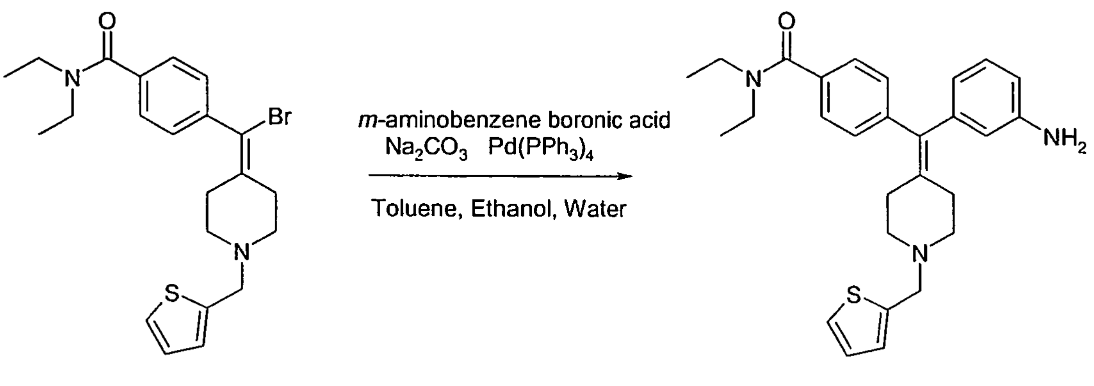


Intermediate 7a

Intermediate 8a

To a solution of INTERMEDIATE 7a (1.702 g, 3.81 mmol) in a mixture of toluene (40 ml) and ethanol (S ml) was added *m*-aminobenzene boronic acid monohydrate (0.886 g, 5.71 mmol) and aqueous sodium carbonate (2M, 4.76 ml, 9.52 mmol). Nitrogen was then bubbled in the solution for 25 min prior to the addition of the palladium tetrakistriphenylphosphine (0.439 g, 0.38 mmol). The solution was heated for 5 hours at 90°C then was cooled and saturated ammonium chloride (40 ml) and ethyl acetate were added. The aqueous layer was extracted with two portions of ethyl acetate and the combined organic extracts were dried over anhydrous sodium sulfate, filtered and concentrated. The resulting material was purified by flash chromatography, eluting 5% methanol in dichloromethane to afford INTERMEDIATE 8a as a yellow foam (1.605g, 91%)

COMPOUND 1: *N,N*-diethyl-4-{{3-[(methylsulfonyl)amino]phenyl}[1-(thien-2-ylmethyl)piperidin-4-ylidene]methyl}benzamide

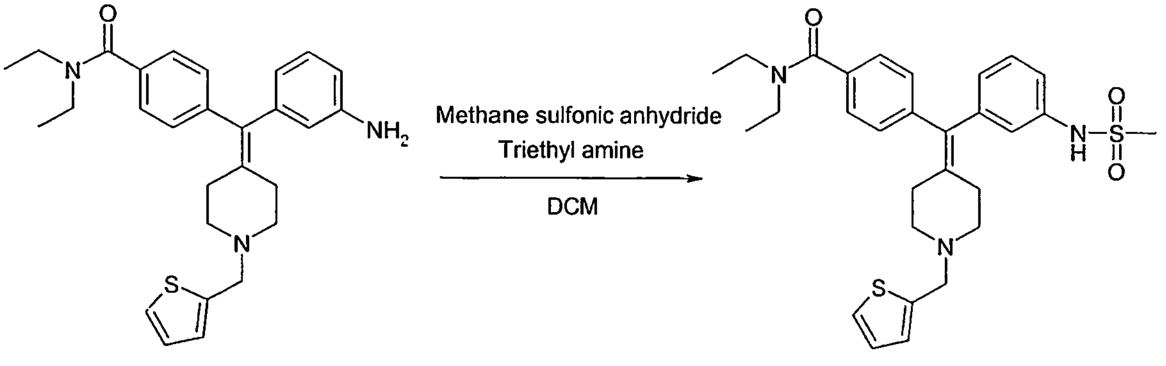


Intermediate 8a

Compound 1

To a solution of INTERMEDIATE 8a (325 mg, 0.7 mmol) in dichloromethane (10 ml) was added triethylamine (302 µl, 0.78 mmol) followed by methane sulfonic anhydride (135mg 2.17 mmol). The solution was stirred for one hour then saturated aqueous sodium bicarbonate (10 ml) was added. The aqueous layer was extracted with two portions of dichloromethane and the combined organic extracts were dried over anhydrous sodium sulfate, filtered and concentrated. The residue was purified by reverse phase chromatography eluting 10% to 40% acetonitrile in water containing 0.1% trifluoroacetic acid. The product was obtained as the trifluoroacetic acid salt and was lyophilized to give a white solid (152mg, 33%). Purity (HPLC): >99% (215nm); >99% (254nm); >99% (280nm). Found: C, 53.83; H, 5.18; N, 6.13. $C_{29}H_{35}N_3O_3S_2$ x $1.5CF_3CO_2H$ x $0.3H_2O$ has C, 53.82; H, 5.24; N, 5.88 %. $^1H$ NMR (400 MHz, $CDCl_3$) δ 1.10-1.15 (m, 3H), 1.22-1.28 (m, 3H), 2.65-2.81 (m, 6H), 2.95 (s, 3H), 3.28 (br s, 2H), 3.54-3.62 (m, 4H), 4.44 (s, 2H), 6.84 (d, J = 7.68 Hz, 1H), 7.01(br s, 1H), 7.04-7.10 (m, 3H), 7.13-7.19 (m, 1H), 7.20-7.23 (m, 1H), 7.30 (d, J = 8.25 Hz, 2H), 7.44 (d, J = 4.96 Hz, 1H), 7.56 (br s, 1H).

INTERMEDIATE 7b: 4- {bromo[1-(2-furylmethyl)piperidin-4-ylidene]methyl}-*N,N*-diethylbenzamide

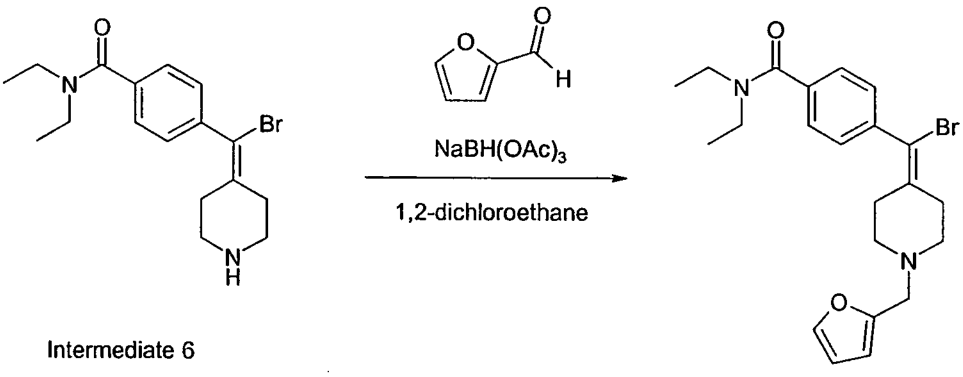


Intermediate 6

Intermediate 7b

To a solution of INTERMEDIATE 6 (1.4g, 3.99mmol) in 1,2-dichloroethane (30ml) was added 2-furaldehyde (62µl, 7.99mmol) and sodium triacetoxyborohydride (1.694g, 7.99mmol). The reaction was stirred at room temperature under nitrogen. After 18 hours the reaction was diluted with dichloromethane and washed with saturated aqueous sodium bicarbonate. The aqueous layer was extracted with two portions of dichloromethane and the combined organic extracts were dried over anhydrous sodium sulfate, filtered and concentrated. The resulting material was purified by flash chromatography, eluting ethyl acetate/hexanes (7:3) to afford INTERMEDIATE 7b (1.503g, 87%) as a pale yellow oil.

INTERMEDIATE 8b: 4-{(3-aminophenyl)[1-(2-%rylmethyl)piperidin-4-ylidene]methyl}-*N,N*-diethylbenzamide

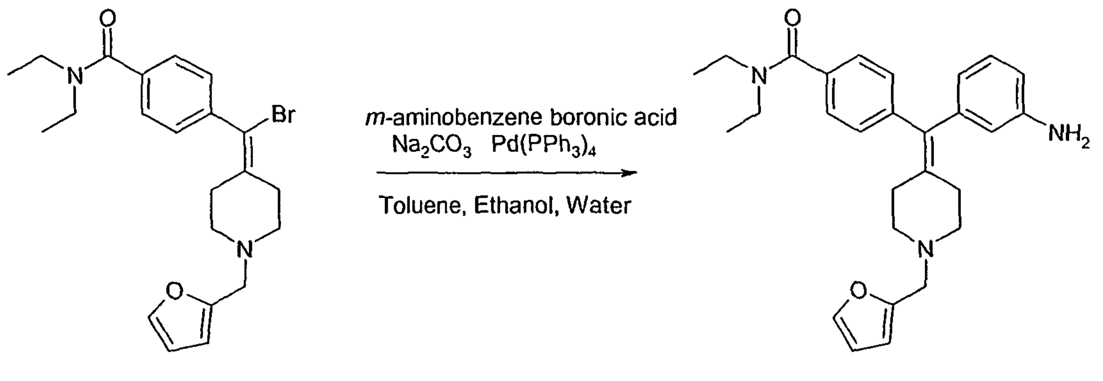


Intermediate 7b

Intermediate 8b

To a solution of INTERMEDIATE 7b (2.120 g, 4.93 mmol) in a mixture of toluene (50 ml) and ethanol (10 ml) was added *m*-aminobenzene boronic acid monohydrate (1.145 g, 7.39 mmol) and aqueous sodium carbonate (2M, 6.15 ml, 12.31 mmol). Nitrogen was then bubbled in the solution for 25 min prior to the addition of the palladium tetrakistriphenylphosphine (0.569 g, 0.49 mmol). The solution was heated for 5 hours at 90°C then was cooled and saturated ammonium chloride (40 ml) and ethyl acetate were added. The aqueous layer was extracted with two portions of ethyl acetate and the combined organic extracts were dried over anhydrous sodium sulfate, filtered and concentrated. The resulting material was purified by flash chromatography, eluting 5% methanol in dichloromethane to afford INTERMEDIATE 8b as a yellow foam (1.967g, 90%).

COMPOUND 2: *N,N*-diethyl-4-[[1-(2-furanylmethyl)-4-piperidinylidene][3-[(methylsulfonyl)amino]phenyl]methyl]-benzamide

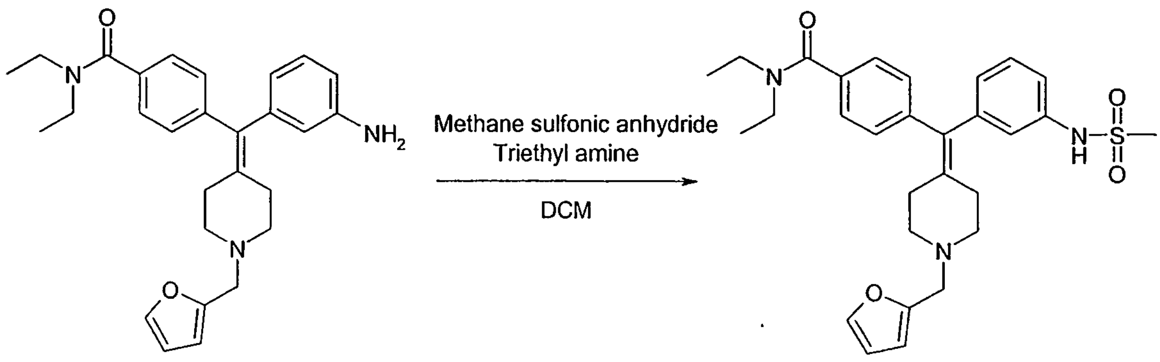


Intermediate 8b

Compound 2

To a solution of INTERMEDIATE 8b (427 mg, 0.96 mmol) in dichloromethane (10 ml) was added triethylamine (416 µl, 2.97 mmol) followed by methane sulfonic anhydride (184 mg 1.06 mmol). The solution was stirred for one hour then saturated sodium bicarbonate (10 ml) was added. The aqueous layer was extracted with two portions of dichloromethane and the combined organic extracts were dried over anhydrous sodium sulfate, filtered and concentrated. The residue was purified by reverse phase chromatography, eluting 10% to 40% acetonitrile in water containing 0.1% trifluoroacetic acid. The product was repurified by flash chromatography, eluting 1% ammonium hydroxide 10% methanol in dichloromethane. The product was dissolved in diethyl ether (15 ml) and a solution of 1M HCl in diethyl ether (2 ml) was added and the solvent evaporated to afford COMPOUND 2 as the corresponding HCl salt and as a white powder (128 mg; 23% yield). Purity (HPLC): >99% (215nm); >99% (254nm); >99% (280nm). Found: C, 59.67; H, 6.58; N, 7.18.

$C_{29}H_{35}N_3O_4S$ x 1.2HCl x 1.0$H_2O$ has C, 59.70; H, 6.60 N, 7.20 % $^1$H NMR (400 MHz, $CDCl_3$) δ 1.12-1.24 (m, 6H), 1.87 (br s, 2H), 2.68 (br s, 2H), 2.98 (s, 3H), 3.00 (br s, 2H), 3.27 (br s, 2H), 3.45-3.60 (m, 4H), 4.29 (br s, 2H), 6.46 (br s, 1H), 6.80 (br s, 2H), 7.08 (br s, 2H), 7.14 (br s, 1H), 7.26 br s, 2H), 7.30 (br s, 2H), 7.51 (br s, 1H).

INTERMEDIATE 7c: 4-{bromo[1-(phenylmethyl)piperidin-4-ylidene]methyl}-*N, N*- diethylbenzamide

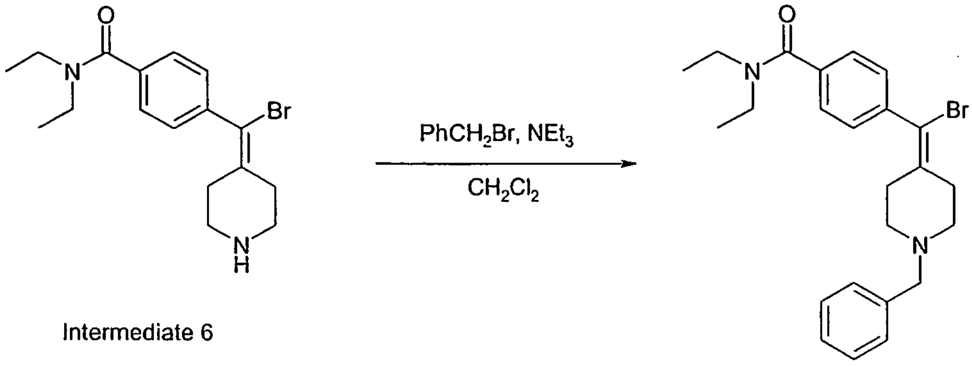


Intermediate 6

Intermediate 7c

To a solution of INTERMEDIATE 6 (7.783g, 22.2mmol) in dichloromethane (160ml) was added triethyl amine (9.3mL, 66.8mmol) and benzyl bromide (3.2mL, 26.9mmol). The reaction was stirred at room temperature under nitrogen. After 24 hours the reaction was washed with water and the aqueous layer was extracted with dichloromethane. The combined organic extracts were dried over anhydrous sodium sulfate, filtered and concentrated. The resulted material was purified by flash chromatography, eluting ethyl acetate/hexanes (7:3) to afford INTERMEDIATE 7c (6.89g, 70%) as colourless solid.

INTERMEDIATE 8c: 4-{(3-aminophenyl)[1-(phenylmethyl)piperidin-4-ylidene]methyl}-*N,N*-diethylbenzamide

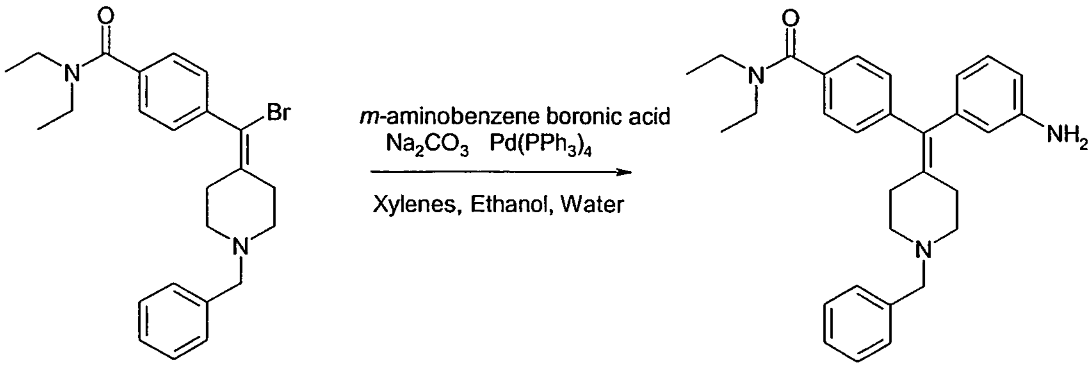


Intermediate 7c

Intermediate 8c

To a solution of INTERMEDIATE 7c (8.50 g, 19.3 mmol) in a mixture of xylenes (120 ml) and ethanol (80 ml) was added *m*-aminobenzene boronic acid monohydrate (3.96 g, 28.9 mmol) and aqueous sodium carbonate (2M, 29.0 ml, 58 mmol). Nitrogen was then bubbled in the solution for 25 min prior to the addition of the palladium tetrakistriphenylphosphine (1.67 g, 1.4 mmol). The solution was heated for 18 hours at 90°C then was cooled and water (60 ml) and ethyl acetate were added. The aqueous layer was extracted with two portions of ethyl acetate and the combined organic extracts were dried over anhydrous sodium sulfate, filtered and concentrated. The resulted material was purified by flash chromatography, eluting 2% to 4% methanol in dichloromethane to afford INTERMEDIATE 8c as an orange foam (8.14g,

93%).

COMPOUND 3: *N,N*-diethyl-4-[[1-(phenylmethyl)-4-pipelidinylidene][3-[(methylsulfonyl)amino]phenyl]methyl]-benzamide

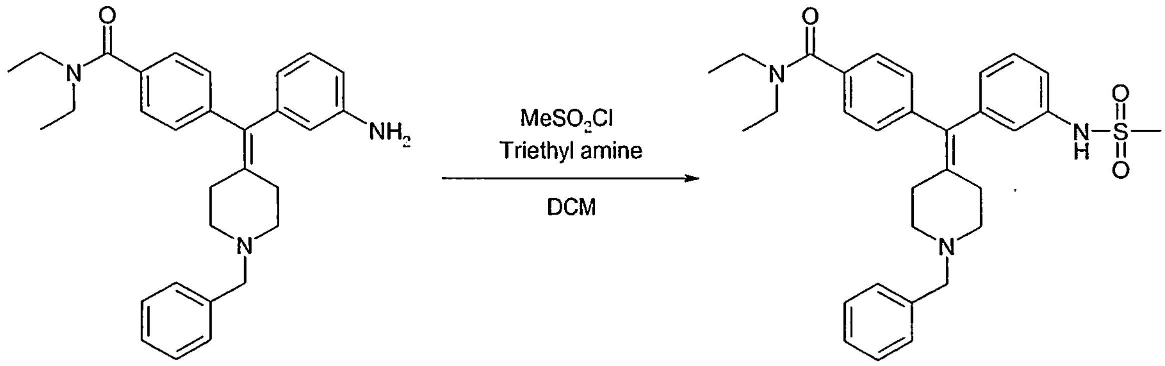


Intermediate 8c

Compound 3

To a solution of INTERMEDIATE 8c (392 mg, 0.87 mmol) in dichloromethane (15 ml) was added triethylamine (145 μl, 1.04 mmol) followed by methane sulfonyl chloride (80μl, 1.03mmol). The solution was stirred for four hours then saturated sodium bicarbonate (10 ml) was added. The aqueous layer was extracted with two portions of dichloromethane and the combined organic extracts were dried over anhydrous sodium sulfate, filtered and concentrated. The residue was purified by flash chromatography, eluting 3% methanol in dichloromethane. The residue was further purified by reverse phase chromatography, eluting 20% to 50% acetonitrile in water containing 0.1% trifluoroacetic acid. COMPOUND 3, obtained as the trifluoroacetic acid salt, was lyophilized to give a colourless solid (170.4mg, 44% yield). Purity (HPLC): >99% (215nm); >99% (254nm); >99% (280mn). Found: C, 58.9; H, 5.61; N, 6.15. $C_{31}H_{37}N_3O_3S$ x 1.3TFA x $0.3H_2O$ has C, 58.9;H, 5.72; N, 6.13%. $^1H$ NMR (400MHz, $CD_3OD$) δ 1.10 (t, J = 6.5Hz, 3H), 1.22 (t, J = 6.5Hz, 3H), 2.48-2.51 (m, 2H), 2.72-2.83 (m, 2H), 2.89 (s, 3H), 3.06-3.11 (m, 2H), 3.27-3.29 (m, 2H), 3.50-3.53 (m, 4H), 4.34 (s, 2H), 6.91 (d, J = 7.5Hz, 1H), 7.09-7.11 (m, 2H), 7.24 (d, J = 8.5Hz, 2H), 7.28-7.36 (m, 3H), 7.49 (s, 5H).

INTERMEDIATE 7d: 4-[bromo[1-(3-pyridinylmethyl)-4-piperidinylidene]methyl]-*N,N*-diethyl-benzamide

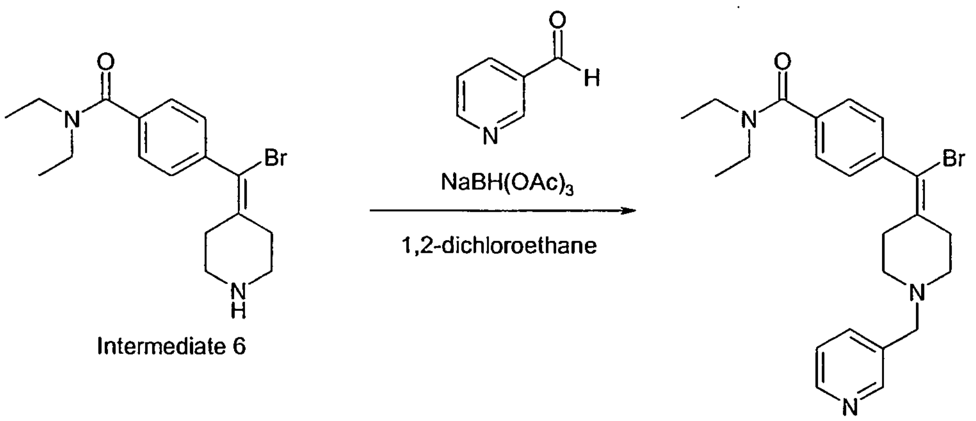


Intermediate 6

Intermediate 7d

To a solution of INTERMEDIATE 6 (0.5g, 1.42mmol) in 1,2-dichloroethane (15ml) was added 3-pyridine carboxaldehyde (160μl, 1.71mmol) and sodium triacetoxyborohydride (392mg, 1.85mmol). The reaction was stirred at room

temperature under nitrogen. After 18 hours the reaction was diluted with dichloromethane and washed with saturated aqueous sodium bicarbonate. The aqueous layer was extracted with two portions of dichloromethane and the combined organic extracts were dried over anhydrous sodium sulfate, filtered and concentrated. The resulting material was purified by flash chromatography, eluting 5% methanol in dichloromethane to afford INTERMEDIATE 7d (630mg, 100%) as a yellow oil.

INTERMEDIATE 8d: 4-[(3-aminophenyl)[1-(3-pyridinylmethyl)-4-piperidinylidene]methyl]-*N,N*-diethyl-benzamide

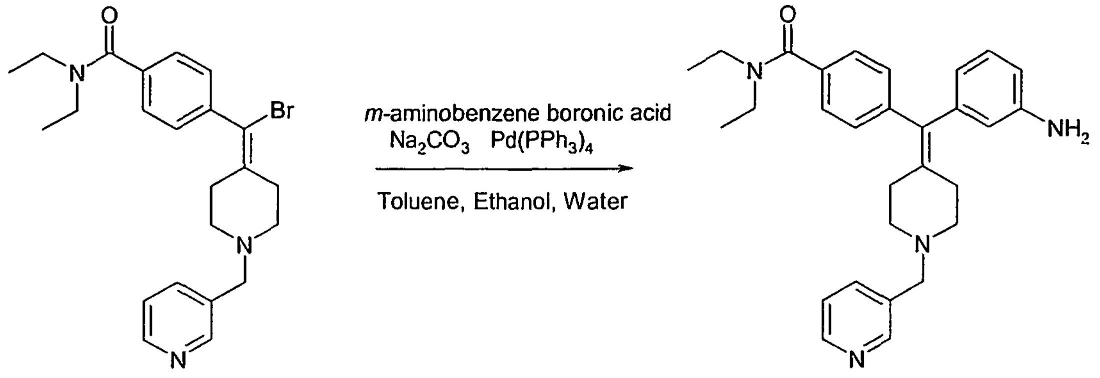


Intermediate 7d

Intermediate 8d

To a solution of INTERMEDIATE 7d (630mg, 1.42 mmol) in a mixture of toluene (9 ml) and ethanol (2 ml) was added *m*-aminobenzene boronic acid monohydrate (264mg, 1.70 mmol) and aqueous sodium carbonate (2M, 1.8 ml, 3.55 mmol). Nitrogen was then bubbled in the solution for 25 min prior to the addition of the palladium tetrakistriphenylphosphine (98mg, 0.09 mmol). The solution was heated for 5 hours at 90°C then was cooled and saturated ammonium chloride (40 ml) and ethyl acetate were added. The aqueous layer was extracted with two portions of ethyl acetate and the combined organic extracts were dried over anhydrous sodium sulfate, filtered and concentrated. The resulted material was purified by flash chromatography, eluting 3% to 5% methanol in dichloromethane to afford INTERMEDIATE 8d as a colourless foam (559mg, 84%).

COMPOUND 4: *N,N*-diethyl-4-[[3-[(methylsulfonyl)amino]phenyl][1-(3-pyridinylmethyl)-4-piperidinylidene]methyl]-benzamide

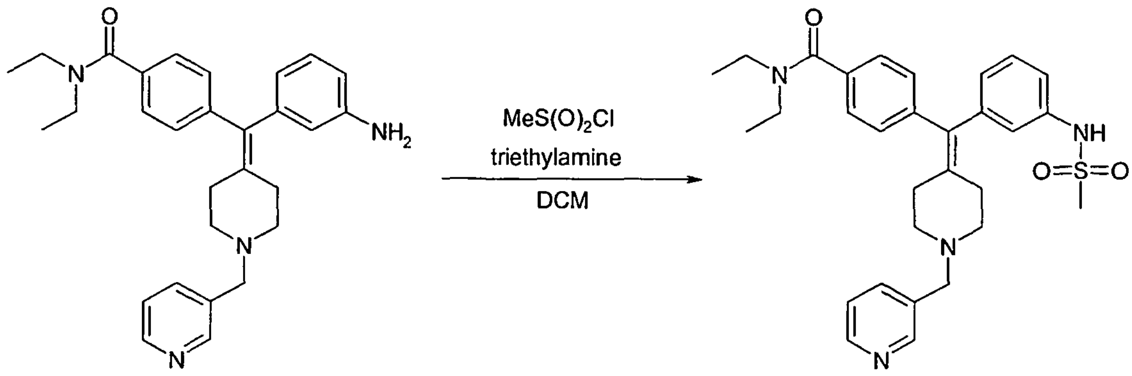


Intermediate 8d

Compound 4

To a solution of INTERMEDIATE 8d (272 mg, 0.60 mmol) in dichloromethane (10 ml) was added triethylamine (290 μl, 2.09 mmol) followed by methane sulfonyl chloride (60μl, 0.77 mmol). The solution was stirred at room temperature for

3 days than saturated sodium bicarbonate (10 ml) was added. The aqueous layer was extracted with two portions of dichloromethane and the combined organic extracts were dried over anhydrous sodium sulfate, filtered and concentrated. The residue was purified by reverse phase chromatography, eluting 10% to 40% acetonitrile in water containing 0.1% trifluoroacetic acid. COMPOUND 4, obtained as the trifluoroacetic acid salt, was lyophilized to give a colourless solid (193.2mg, 43% yield). Purity (HPLC): >99% (215nm); >99% (254nm); >99% (280nm). Found: C, 51.23; H, 4.75; N, 6.93. $C_{30}H_{36}N_4O_3S$ x $0.1H_2O$ x 2.5TFA has C, 51.29; H, 4.76; N, 6.84%. $^1H$ NMR (400MHz, $CD_3OD$) $\delta$ 1.10 (t, J = 6.9 Hz, 3H), 1.22 (t, J = 6.8 Hz, 3H), 2.65 (br s, 4H), 3.26-3.54 (m, 8H), 4.47 (s, 2H), 6.92 (d, J = 7.6 Hz, 1H), 7.10-7.12 (m, 2H), 7.24 (d, J = 8.0 Hz, 2H), 7.29-7.37 (m, 3H), 7.68 (dd, J = 5.1, 8.0 Hz, 1H), 8.14 (d, J = 8.0 Hz, 1H), 8.73-8.77 (m, 2H).

INTERMEDIATE 9: 4-[[4-[(diethylamino)carbonyl]phenyl](3-nitrophenyl)methylene]-1-piperidinecarboxylic acid, 1,1-dimethylethyl ester

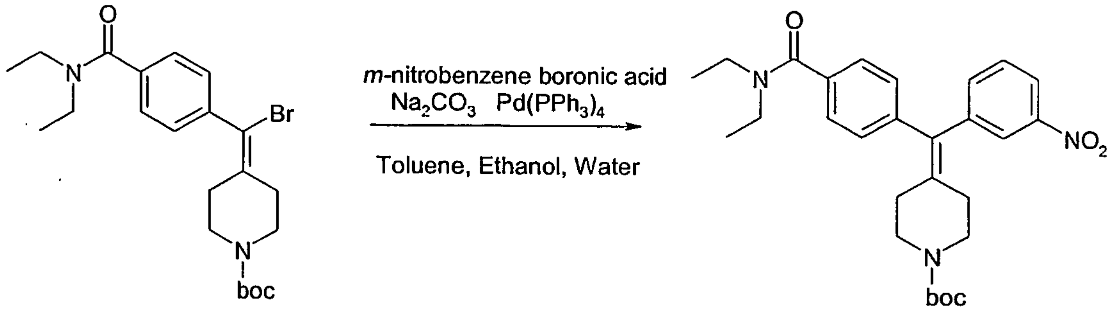


Intermediate 5 Intermediate 9

Synthesized as shown for INTERMEDIATE 8c except that INTERMEDIATE 5 was used as the vinyl bromide and *m*-nitrobenzene boronic acid as the boronic acid.

INTERMEDIATE 10: *N,N*-diethyl-4-[(3-nitrophenyl)-4-piperidinylidenemethyl]-benzamide

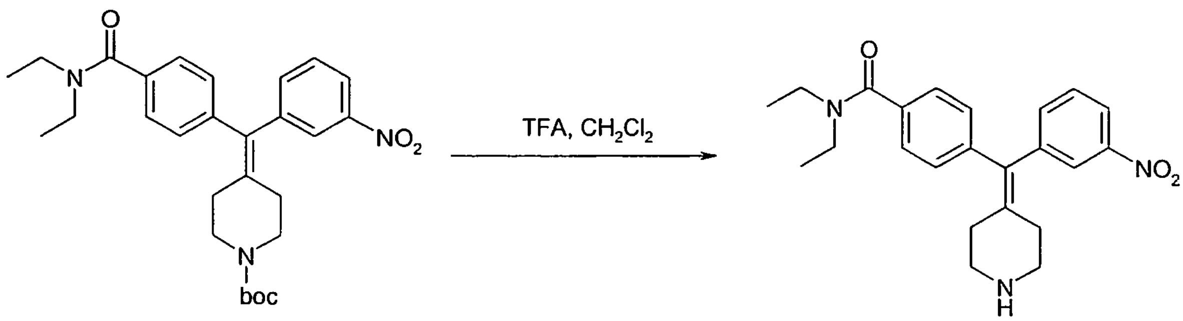


Intermediate 9

Intermediate 10

To a solution of INTERMEDIATE 9 (1.0g, 2.03 mmol) in dichloromethane (15 ml) was added trifluoroacetic acid (1.6 ml, 20.7 mmol). The reaction was stirred overnight at room temperature then was quenched with aqueous sodium hydroxide (2M, 15 ml). The aqueous layer was separated and washed with dichloromethane (20 ml). The combined organic extracts were dried ($MgSO_4$), filtered and concentrated. The residue was purified by flash chromatography, eluting 35% methanol in dichloromethane rising to 50% methanol in dichloromethane to yield INTERMEDIATE 10 (613.5mg, 77%) as a yellow foam.

INTERMEDIATE 11: *N,N*-diethyl-4-{(3-nitrophenyl)[1-(1,3-thiazol-2-ylmethyl)piperidin-4- ylidene]methyl}benzamide.

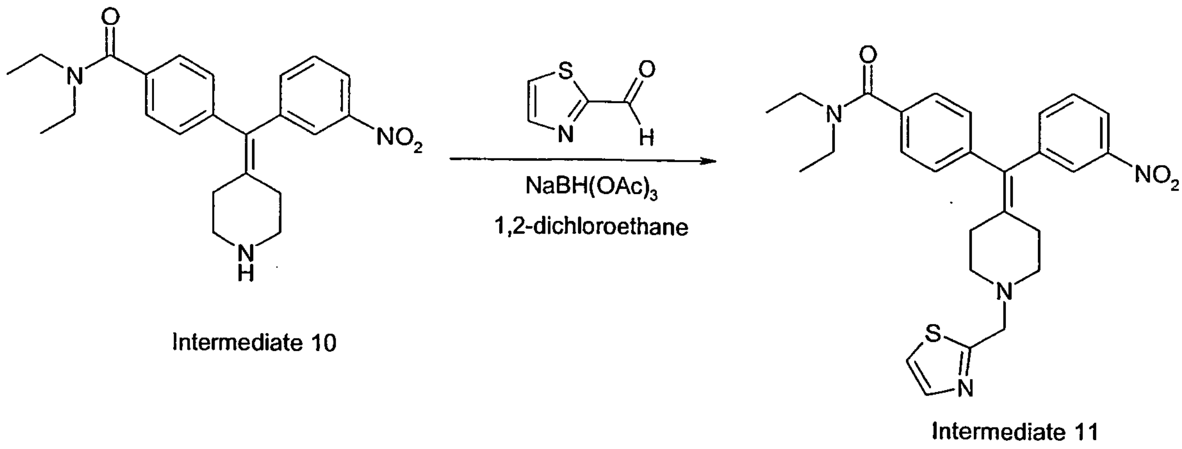


Intermediate 10

Intermediate 11

To a solution of INTERMEDIATE 10 (341mg, 0.87 mmol) in 1,2-dichloroethane (10ml) was added 2-thiazole carboxaldehyde (91μl, 1.71mmol) and sodium triacetoxyborohydride (392mg, 1.04 mmol). The reaction was stirred at room temperature under nitrogen. After 3 days at room temperature the reaction was diluted with dichloromethane and washed with saturated aqueous sodium bicarbonate. The aqueous layer was extracted with two portions of dichloromethane and the combined organic extracts were dried over anhydrous sodium sulfate, filtered and concentrated. The resulting material was purified by flash chromatography, eluting 3% methanol in dichloromethane to afford INTERMEDIATE 11 (332mg, 78%) as a colourless foam.

INTERMEDIATE 12: 4-{(3-aminophenyl)[1-(1,3-thiazol-2-ylmethyl)piperidin-4-ylidene]methyl}-*N,N*-diethylbenzamide

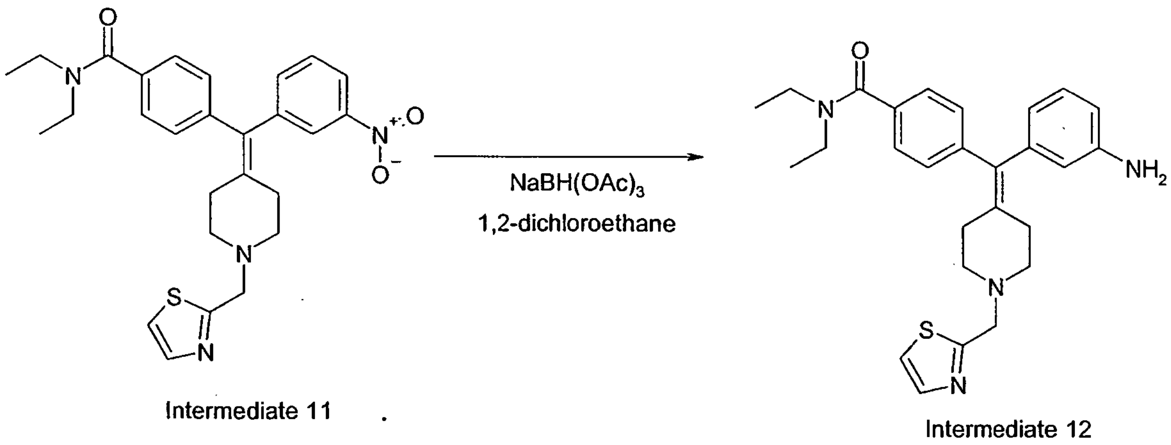


Intermediate 11

Intermediate 12

To a solution of INTERMEDIATE 11 (266mg, 0.54 mmol) in a mixture of ethanol, tetrahydrofuran, water and aqueous saturated ammonium chloride (4:2:1:1 ratio v/v) (3 ml) was added a small amount of iron powder and the reaction was heated for 5 minutes in a microwave at 150 °C. The solids were removed by filtration and the product extracted with ethyl acetate to yield INTERMEDIATE 12 (249.7mg, 100%) as a colourless foam.

COMPOUND 5: *N,N*-diethyl-4-[[3-[(methylsulfonyl)amino]phenyl][1-(3-thiazolylmethyl)-4-piperidinylidene]methyl]-benzamide

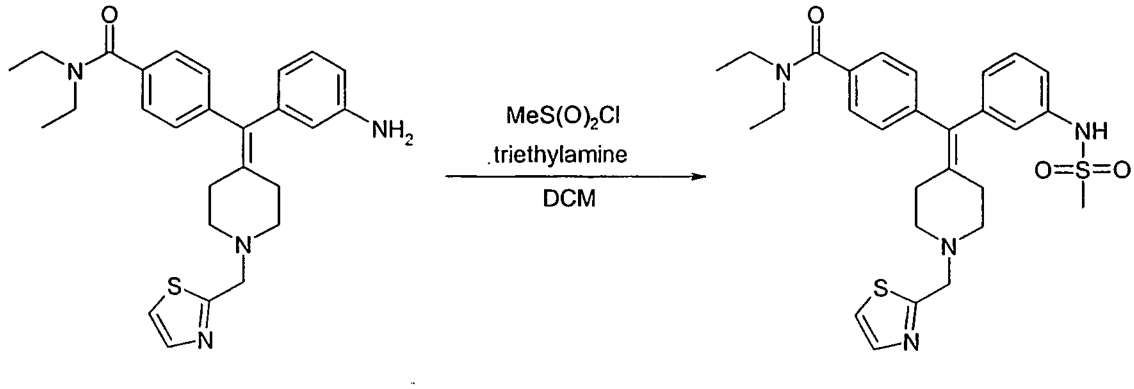


Intermediate 12

Compound 5

To a solution of INTERMEDIATE 12 (171 mg, 0.37 mmol) in dichloromethane (10 ml) was added triethylamine (181 μl, 1.31 mmol) followed by methane sulfonyl chloride (45μl, 0.58 mmol). The solution was stirred at room temperature for 4 days than saturated sodium bicarbonate (10 ml) was added. The aqueous layer was extracted with two portions of dichloromethane and the combined organic extracts were dried over anhydrous sodium sulfate, filtered and concentrated. The residue was purified by reverse phase chromatography, eluting 10% to 40% acetonitrile in water containing 0.1% trifluoroacetic acid. COMPOUND 5, obtained as the trifluoroacetic acid salt, was lyophilized to give a colourless solid (40.7mg, 14% yield). Purity (HPLC): >99% (215nm); >99% (254nm); >99% (280nm). Found: C, 51.23; H, 4.75; N, 6.93. $C_{30}H_{36}N_4O_3S$ x 0.1H2O x 2.5TFA has C, 51.29; H, 4.76; N, 6.84%. NMR (400MHz, $CD_3OD$) δ 1.10 (t, J = 6.9 Hz, 3H), 1.22 (t, J = 6.8 Hz, 3H), 2.65 (br s, 4H), 3.26-3.54 (m, 8H), 4.47 (s, 2H), 6.92 (d, J = 7.6 Hz, 1H), 7.10-7.12 (m, 2H), 7.24 (d, J = 8.0 Hz, 2H), 7.29-7.37 (m, 3H), 7.68 (dd, J = 5.1, 8.0 Hz, 1H), 8.14 (d, J = 8.0 Hz, 1H), 8.73-8.77 (m, 2H).

## Claims

1. A compound of formula I, a pharmaceutically acceptable salt thereof, diastereomers, enantiomers, or mixtures thereof:

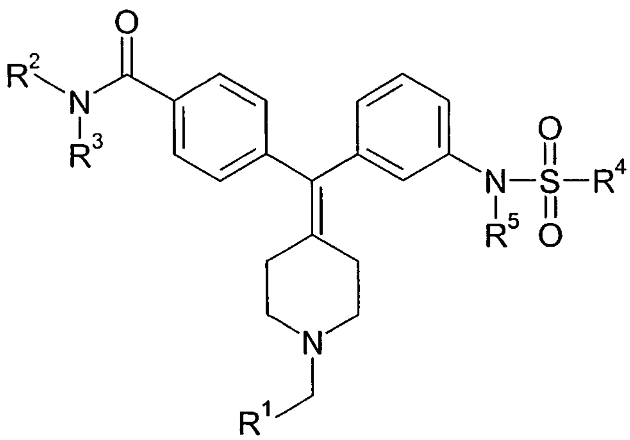

**I**

wherein

$R^1$ is selected from $C_{6-10}$aryl and $C_{2-6}$heteroaryl, wherein said $C_{6-10}$aryl and $C_{2-6}$heteroaryl are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R, and -NRC(=O)-OR, wherein R is, independently, a hydrogen or $C_{1-6}$alkyl; and

$R^2$, $R^3$, $R^4$ and $R^5$ are, independently, selected from hydrogen, $C_{1-6}$alkyl, and $C_{3-6}$cycloalkyl, wherein said

$C_{1-6}$alkyl and $C_{3-6}$cycloalkyl are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH,-C(=O)OR, $-C(=O)NR_2$, -NRC(=O)R, and -NRC(=O)-OR, wherein R is, independently, a hydrogen or $C_{1-6}$alkyl.

**2.** A compound according to claim 1,
wherein $R^1$ is selected from phenyl; pyridyl; thienyl; furyl; imidazolyl; triazolyl; pyrrolyl; thiazolyl; and N-oxido-pyridyl, wherein $R^1$ is optionally substituted with one or more groups selected from $C_{1-6}$alkyl, halogenated $C_{1-6}$alkyl,$-NO_2$, $-CF_3$, $C_{1-6}$ alkoxy, chloro, fluoro, bromo, and iodo;
$R^2$, $R^3$, and $R^4$ are, independently, $C_{1-3}$alkyl or halogenated $C_{1-3}$alkyl;
$R^5$ is selected from hydrogen, $C_{1-6}$alkyl, and $C_{3-6}$cycloalkyl, wherein said $C_{1-6}$alkyl and $C_{3-6}$cycloalkyl are optionally substituted with one or more groups selected from $C_{1-6}$alkyl, halogenated $C_{1-6}$alkyl, $-NO_2$, $-CF_3$, $C_{1-6}$ alkoxy, chloro, fluoro, bromo, and iodo.

**3.** A compound according to claim 1,
wherein $R^1$ is selected from phenyl; pyridyl; thienyl; furyl; imidazolyl; pyrrolyl; and thiazolyl, wherein $R^1$ is optionally substituted with one or more groups selected from $C_{1-6}$alkyl, halogenated $C_{1-6}$alkyl, $-NO_2$, $-CF_3$, $C_{1-6}$ alkoxy, chloro, fluoro, bromo, and iodo;
$R^2$, $R^3$, and $R^4$ are, independently, $C_{1-3}$alkyl or halogenated $C_{1-3}$alkyl; and
$R^5$ is hydrogen.

**4.** A compound according to claim 1,
wherein $R^1$ is selected from phenyl, pyridyl, thienyl, furyl, imidazolyl, pyrrolyl, and thiazolyl;
$R^2$ and $R^3$ are ethyl;
$R^4$ is $C_{1-3}$alkyl; and
$R^5$ is hydrogen.

**5.** A compound according to claim 1, wherein the compound is selected from:

*N,N*-diethyl-4-{{3-[(methylsulfonyl)amino]phenyl}[1-(thien-2-ylmethyl)piperidin-4-ylidene]methyl}benzamide;
*N,N*-diethyl-4-[[1-(2-furanylmethyl)-4-piperidinylidene][3-[(methylsulfonyl)amino]phenyl]methyl]-benzamide;
*N,N*-diethyl-4-[[1-(phenylmethyl)-4-piperidinylidene][3-[(methylsulfonyl)amino]phenyl]methyl]-benzamide;
*N,N*-diethyl-4-[[3-[(methylsulfonyl)amino]phenyl][1-(3-pyridinyhnethyl)-4-piperidinylidene]methyl]-benzamide;
*N,N*-diethyl-4-[[3-[(methylsulfonyl)amino]phenyl][1-(3-thiazolyl-methyl)-4-piperidinylidene]methyl]-benzamide;

and pharmaceutically acceptable salts thereof.

**6.** A compound according to any one of claims 1-5 for use as a medicament.

**7.** The use of a compound according to any one of claims 1-5 in the manufacture of a medicament for the therapy of pain, anxiety or functional gastrointestinal disorders.

**8.** A pharmaceutical composition comprising a compound according to any one of claims 1-5 and a pharmaceutically acceptable carrier.

**9.** A process for preparing a compound of formula I, comprising:

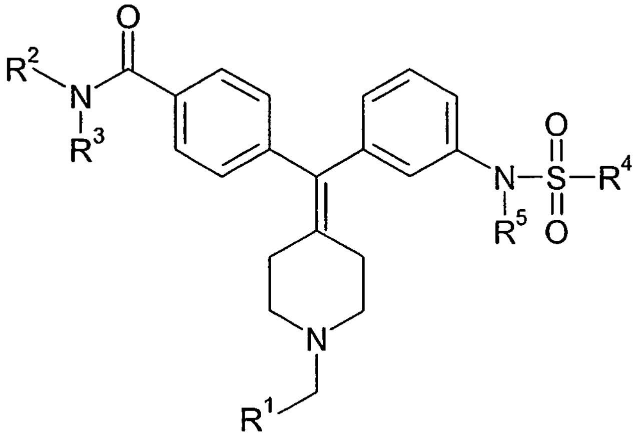

I

reacting a compound of formula II with $X-S(=O)_2-R^4$ or $R^4S(=O)_2-O-S(=O)_2R^4$.

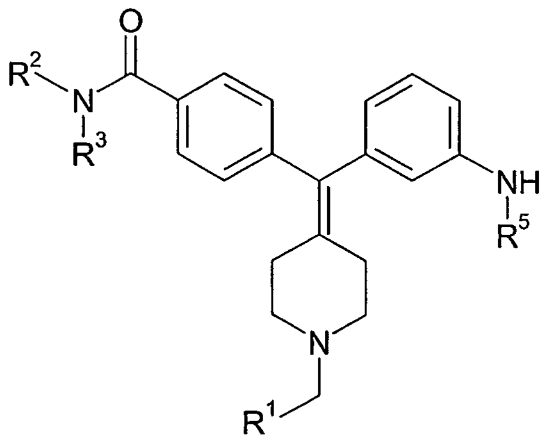

,

II

wherein

X is selected from Cl, Br and I;
$R^1$ is selected from $C_{6-10}$aryl and $C_{2-6}$heteroaryl, wherein said $C_{6-10}$aryl and $C_{2-6}$heteroaryl are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R, and -NRC (=O)-OR, wherein R is, independently, a hydrogen or $C_{1-6}$alkyl; and
$R^2$, $R^3$, $R^4$ and $R^5$ are, independently, selected from hydrogen, $C_{1-6}$akyl, and $C_{3-6}$cycloalkyl, wherein said $C_{1-6}$alkyl and $C_{3-6}$cycloalkyl are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH,-C (=O)OR,- $C(=O)NR_2$, -NRC(=O)R, and -NRC(=O)-OR, wherein R is, independently, a hydrogen or $C_{1-6}$alkyl.

**Patentansprüche**

1. Verbindungen der Formel I, deren pharmazeutisch annehmbare Salze, Diastereomere, Enantiomere oder Mischungen davon:

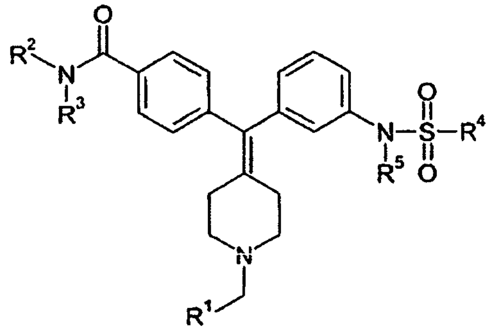

I

wobei

$R^1$ ausgewählt ist aus $C_{6-10}$-Aryl und $C_{2-6}$-Heteroaryl, wobei diese $C_{6-10}$-Aryl- und $C_{2-6}$-Heteroarylreste gegebenenfalls durch eine oder mehrere Gruppen ausgewählt aus -R, $-NO_2$, -OR, -Cl, -Br, -I, -F, $-CF_3$, -C(=O)R, -C(=O)OH, $-NH_2$, -SH, -NHR, $-NR_2$,-SR, $-SO_3H$, $-SO_2R$, -S(=O)R, -CN, -OH, -C(=O)OR,-C(=O)$NR_2$, -NRC(=O)R und -NRC(=O)-OR substituiert sind, wobei die Reste R unabhängig voneinander für Wasserstoff oder $C_{1-6}$-Alkyl stehen; und

$R^2$, $R^3$, $R^4$ und $R^5$ unabhängig voneinander ausgewählt sind aus Wasserstoff, $C_{1-6}$-Alkyl und $C_{3-6}$-Cycloalkyl, wobei die $C_{1-6}$-Alkyl- und $C_{3-6}$-Cyckloalkylreste gegebenenfalls durch eine oder mehrere Gruppen ausgewählt aus -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, -C(=O)$NR_2$, -NRC(=O)R und -NRC(=O)-OR substituiert sind, wobei die Reste R unabhängig voneinander für Wasserstoff oder $C_{1-6}$-Alkyl stehen.

**2.** Verbindungen nach Anspruch 1,
wobei $R^1$ ausgewählt ist aus Phenyl, Pyridyl, Thienyl, Furyl, Imidazolyl, Triazolyl, Pyrrolyl, Thiazolyl und N-Oxido-pyridyl, wobei $R^1$ gegebenenfalls durch eine oder mehrere Gruppen ausgewählt aus $C_{1-6}$-Alkyl, halogeniertem $C_{1-6}$-Alkyl, $-NO_2$, $-CF_3$, $C_{1-6}$-Alkoxy, Fluor, Chlor, Brom und Iod substituiert ist;
$R^2$, $R^3$ und $R^4$ unabhängig voneinander für $C_{1-3}$-Alkyl oder halogeniertes $C_{1-3}$-Alkyl stehen;
$R^5$ ausgewählt ist aus Wasserstoff, $C_{1-6}$-Alkyl und $C_{3-6}$-Cycloalkyl, wobei die $C_{1-6}$-Alkyl- und $C_{3-6}$-Cycloalkylreste gegebenenfalls durch eine oder mehrere Gruppen ausgewählt aus $C_{1-6}$-Alkyl, halogeniertem $C_{1-6}$-Alkyl, $-NO_2$, $-CF_3$, $C_{1-6}$-Alkoxy, Chlor, Fluor, Brom und Iod substituiert sind.

**3.** Verbindungen nach Anspruch 1,
wobei $R^1$ ausgewählt ist aus Phenyl, Pyridyl, Thienyl, Furyl, Imidazolyl, Pyrrolyl und Thiazolyl, wobei $R^1$ gegebenenfalls durch eine oder mehrere Gruppen ausgewählt aus $C_{1-6}$-Alkyl, halogeniertem $C_{1-6}$-Alkyl, $-NO_2$, $-CF_3$, $C_{1-6}$-Alkoxy, Chlor, Fluor, Brom und Iod substituiert ist;
$R^2$, $R^3$ und $R^4$ unabhängig voneinander für $C_{1-3}$-Alkyl oder halogeniertes $C_{1-3}$-Alkyl stehen; und
$R^5$ für Wasserstoff steht.

**4.** Verbindungen nach Anspruch 1,
wobei $R^1$ ausgewählt ist aus Phenyl, Pyridyl, Thienyl, Furyl, Imidazolyl, Pyrrolyl und Thiazolyl;
$R^2$ und $R^3$ für Ethyl stehen;
$R^4$ für $C_{1-3}$-Alkyl steht; und
$R^5$ für Wasserstoff steht.

**5.** Verbindungen nach Anspruch 1, ausgewählt aus:

*N,N*-Diethyl-4-{{3-[(methylsulfonyl]amino]phenyl}[1-(thien-2-ylmethyl)piperidin-4-yliden]methyl}benzamid;
*N,N*-Diethyl-4-[[1-(2-furanylmethyl)-4-piperidinyliden][3-[(methylsulfonyl)amino]phenyl]methyl]benzamid;
*N,N*-Diethyl-4-[[1-(phenylmethyl)-4-piperidinyliden][3-[(methylsulfonyl)amino]phenyl]methyl]benzamid;
*N,N*-Diethyl-4-[[3-[(methylsulfonyl]amino]phenyl][1-(3-pyridinylmethyl)-4-piperidinyliden]methyl]benzamid;
*N,N*-Diethyl-4-[[3-[(methylsulfonyl]amino]phenyl][1-(3-thiazolylmethyl)-4-piperidinyliden]methyl]benzamid;

und deren pharmazeutisch annehmbaren Salze.

**6.** Verbindungen nach einem der Ansprüche 1-5 zur Verwendung als Medikament.

**7.** Verwendung einer Verbindung nach einem der Ansprüche 1-5 bei der Herstellung eines Medikaments für die Therapie von Schmerzen, Ängstlichkeit oder funktionellen gastrointestinalen Erkrankungen.

**8.** Pharmazeutische Zusammensetzung, enthaltend eine Verbindung nach einem der Ansprüche 1-5 und einen pharmazeutisch annehmbaren Träger.

**9.** Verfahren zur Herstellung einer Verbindung der Formel I, bei dem man:

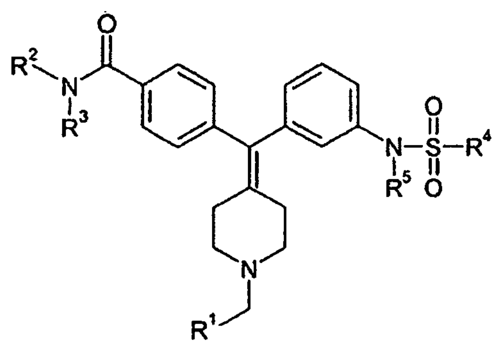

I

eine Verbindung der Formel II mit $X\text{-}S(=O)_2\text{-}R^4$ oder $R^4S(=O)_2\text{-}O\text{-}S(=O)_2R^4$ umsetzt,

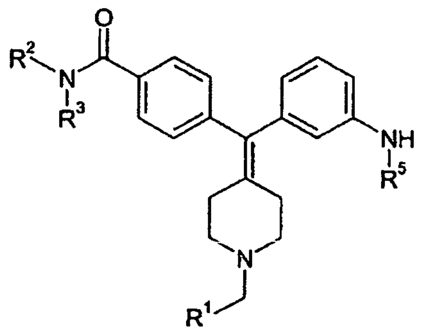

,

II

wobei

X ausgewählt ist aus Cl, Br und I;

$R^1$ ausgewählt ist aus $C_{6\text{-}10}$-Aryl und $C_{2\text{-}6}$-Heteroaryl, wobei diese $C_{6\text{-}10}$-Aryl- und $C_{2\text{-}6}$-Heteroarylreste gegebenenfalls durch eine oder mehrere Gruppen ausgewählt aus -R, $-NO_2$, -OR, -Cl, -Br, -I, -F, $-CF_3$, -C(=O)R, -C(=O)OH, $-NH_2$, -SH, -NHR, $-NR_2$,-SR, $-SO_3H$, $-SO_2R$, -S(=O)R, -CN, -OH, -C(=O)OR,$-C(=O)NR_2$, -NRC(=O)R und -NRC(=O)-OR substituiert sind, wobei die Reste R unabhängig voneinander für Wasserstoff oder $C_{1\text{-}6}$-Alkyl stehen; und

$R^2$, $R^3$, $R^4$ und $R^5$ unabhängig voneinander ausgewählt sind aus Wasserstoff, $C_{1\text{-}6}$-Alkyl und $C_{3\text{-}6}$-Cycloalkyl, wobei die $C_{1\text{-}6}$-Alkyl- und $C_{3\text{-}6}$-Cycloalkylreste gegebenenfalls durch eine oder mehrere Gruppen ausgewählt aus -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R und -NRC(=O)-OR substituiert sind, wobei die Reste R unabhängig voneinander für Wasserstoff oder $C_{1\text{-}6}$-Alkyl stehen.

## Revendications

**1.** Composé de formule I, un sel pharmaceutiquement acceptable de celui-ci, les diastéréoisomères, les énantiomères ou les mélanges de celui-ci :

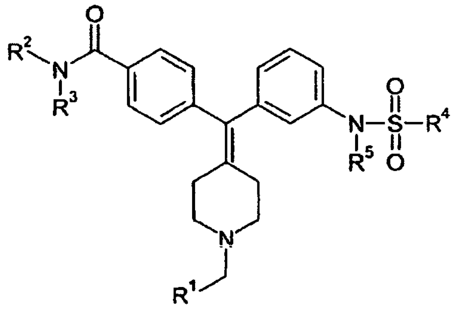

I

dans laquelle

$R^1$ est choisi parmi $C_{6-10}$-aryle et $C_{2-6}$-hétéroaryle, où lesdits $C_{6-10}$-aryle et $C_{2-6}$-hétéroaryle sont éventuellement substitués par un ou plusieurs groupes choisis parmi -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R et -NRC (=O)-OR où R est indépendamment un hydrogène ou $C_{1-6}$-alkyle ; et

$R^2$, $R^3$, $R^4$ et $R^5$ sont choisis indépendamment parmi hydrogène, $C_{1-6}$-alkyle et $C_{3-6}$-cycloalkyle, où lesdits $C_{1-6}$-alkyle et $C_{3-6}$-cycloalkyle sont éventuellement substitués par un ou plusieurs groupes choisis parmi -R, $-NO_2$, -OR, -Cl, -Br, -I,- F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R et -NRC(=O)-OR où R est indépendamment un hydrogène ou $C_{1-6}$-alkyle.

**2.** Composé selon la revendication 1,
**caractérisé en ce que** $R^1$ est choisi parmi phényle, pyridyle, thiényle, furyle, imidazolyle, triazolyle, pyrrolyle, thiazolyle et N-oxidopyridyle, où $R^1$ est éventuellement substitué par un ou plusieurs groupes choisis parmi $C_{1-6}$alkyle, $C_{1-6}$alkyle halogéné, $-NO_2$, $-CF_3$, $C_{1-6}$alcoxy, chloro, fluoro, bromo et iodo ;
$R^2$, $R^3$ et $R^4$ sont indépendamment $C_{1-3}$alkyle ou $C_{1-3}$alkyle halogéné ;
$R^5$ est choisi parmi hydrogène, $C_{1-6}$alkyle et $C_{3-6}$cycloalkyle, où lesdits $C_{1-6}$alkyle et $C_{3-6}$cycloalkyle sont éventuellement substitués par un ou plusieurs groupes choisis parmi $C_{1-6}$alkyle, $C_{1-6}$alkyle halogéné, $-NO_2$, $-CF_3$, $C_{1-6}$alcoxy, chloro, fluoro, bromo et iodo.

**3.** Composé selon la revendication 1,
**caractérisé en ce que** $R^1$ est choisi parmi phényle, pyridyle, thiényle, furyle, imidazolyle, pyrrolyle et thiazolyle, où $R^1$ est éventuellement substitué par un ou plusieurs groupes choisis parmi $C_{1-6}$alkyle, $C_{1-6}$alkyle halogéné, $-NO_2$, $-CF_3$, $C_{1-6}$alcoxy, chloro, fluoro, bromo et iodo ;
$R^2$, $R^3$ et $R^4$ sont indépendamment $C_{1-3}$alkyle ou $C_{1-3}$alkyle halogéné ; et
$R^5$ est hydrogène.

**4.** Composé selon la revendication 1,
**caractérisé en ce que** $R^1$ est choisi parmi phényle, pyridyle, thiényle, furyle, imidazolyle, pyrrolyle et thiazolyle ;
$R^2$ et $R^3$ sont éthyle ;
$R^4$ est $C_{1-3}$alkyle ; et
$R^5$ est hydrogène.

**5.** Composé selon la revendication 1, **caractérisé en ce que** le composé est choisi parmi :

le *N,N*-diéthyl-4-{{3-[(méthylsulfonyl)amino]-phényl}[1-(thién-2-ylméthyl)pipéridin-4-ylidène]méthyl}benzamide ;
le *N,N*-diéthyl-4-[[1-(2-furanylméthyl)-4-pipéridinylidène][3-[(méthylsulfonyl)amino]phényl]méthyl]benzamide ;
le *N,N*-diéthyl-4-[[1-(phénylméthyl)-4-pipéridinylidène][3-[(méthylsulfonyl)amino]phényl]méthyl]benzamide ;

le *N,N*-diéthyl-4-[[3-[(méthylsulfonyl)amino]-phényl][1-(3-pyridinylméthyl)-4-pipéridinylidène]méthyl]benzamide ;
le *N,N*-diéthyl-4-[[3-[(méthylsulfonyl)amino]-phényl] [1-(3-thiazolylméthyl)-4-pipéridinylidène]méthyl] benzamide ;

et les sels pharmaceutiquement acceptables de ceux-ci.

**6.** Composé selon l'une quelconque des revendications 1-5, destiné à être utilisé comme médicament.

**7.** Utilisation d'un composé selon l'une quelconque des revendications 1-5, dans la fabrication d'un médicament destiné à la thérapie de la douleur, de l'anxiété ou des troubles gastro-intestinaux fonctionnels.

**8.** Composition pharmaceutique comprenant un composé selon l'une quelconque des revendications 1-5 et un support pharmaceutiquement acceptable.

**9.** Procédé de préparation d'un composé de formule I, comprenant :

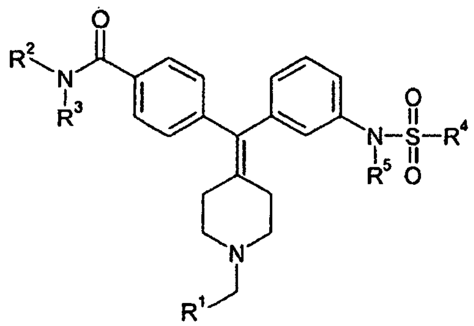

I

la réaction d'un composé de formule II avec X-$S(=O)_2$-$R^4$ ou $R^4S(=O)_2$-O-$S(=O)_2R^4$,

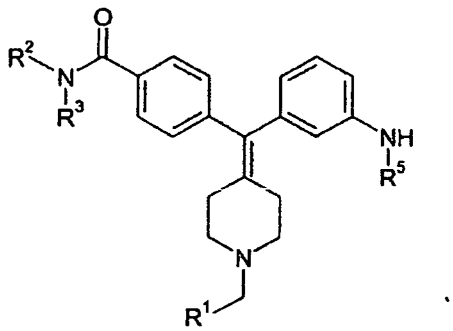

,

II

dans lesquelles

X est choisi parmi Cl, Br et I ;
$R^1$ est choisi parmi $C_{6-10}$-aryle et $C_{2-6}$-hétéroaryle, où lesdits $C_{6-10}$-aryle et $C_{2-6}$-hétéroaryle sont éventuellement substitués par un ou plusieurs groupes choisis parmi -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R et -NRC(=O)-OR où R est indépendamment un hydrogène ou $C_{1-6}$-alkyle ; et
$R^2$, $R^3$, $R^4$ et $R^5$ sont choisis indépendamment parmi hydrogène, $C_{1-6}$-alkyle et $C_{3-6}$-cycloalkyle, où lesdits $C_{1-6}$-alkyle et $C_{3-6}$-cycloalkyle sont éventuellement substitués par un ou plusieurs groupes choisis parmi -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R et -NRC(=O)-OR où R est indépendamment un hydrogène ou $C_{1-6}$-alkyle.